# EUROPEAN PATENT APPLICATION

(11) **EP 1 323 419 A1**
(43) Date of publication of application: **02.07.2003**
(21) Application number: 01972600.9
(22) Date of filing: 28.09.2001
(51) Int. Cl.: A61K 31/4245, A61K 31/4355, A61K 31/4365, A61K 31/437, A61K 31/4439, A61K 31/4725, A61K 31/513, A61K 31/5377, A61K 31/55, A61K 38/05, A61P 11/06, C07D 413/12

(54) **AIRWAY MUCUS SECRETION INHIBITORS**

(30) Priority: 29.09.2000 JP 2000297878
(71) Applicant: ONO PHARMACEUTICAL CO., LTD., Osaka-shi, Osaka 541-8526 (JP)
(72) Inventor: NAGAI, Atsushi, Setagaya-ku, Tokyo 156-0044 (JP); SHIRAJI, Takamitsu, c/o ONO PHARMACEUTICAL CO.,LTD, Osaka-shi, Osaka 541-8526 (JP)
(74) Representative: Henkel, Feiler, Hänzel
(86) International application number: JP0108569
(87) International publication number: WO02026229

(57) **Abstract**

Prophylactic and/or therapeutic drugs against diseases with the augmentative airway mucus secretion, which include five-member heterocyclic compounds represented by the formula (I) or these nontoxic salts as active principle, 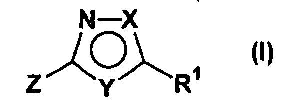 (wherein Z represents the group containing α-aminocarbonyl group, R¹ represents (1) substituted alkyl, alkenyl or alkynyl groups, (2)OH, (3)amino, (4)alkylamino, (5)dialkylamino, etc., X and Y represent oxygen atom, sulfur atom or substituted nitrogen atom.)

## Description

### TECHNICAL FIELD

The present invention relates to restrainers of airway mucus secretion.

More particularly, the present invention relates to, 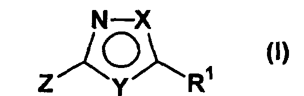 restrainers of airway mucus secretion, which include five-member heterocyclic compounds represented by the formula (I) (all signs in the formula represent the same meanings as postscripts.) and have inhibitory activity against elastase or these nontoxic salts as active principle.

### BACKGROUND OF THE INVENTION

Human neutrophil elastase (HNE) is a kind of serine protease, and protein proteinase that is secreted from neutrophils by various inflammatory stimulus and takes a part in connective tissue biolysis. HNE activity is regulated by alpha1-proteinase inhibitor that is inhibitory factor in vivo, and the symptom of tissue destruction appears when unbalance between the enzyme and the inhibitor arise. For example, it is well known that it is involved in pulmonary emphysema, atherosclerosis and arthritis ,etc.

On the other side, asthma is said as the hypersensitivity reaction that is caused by a certain specific stimulus (antigen) and the obliterative condition of airway that is caused by the reaction. Allergy, abnormality of autonomic nerve, infection, etc. are given as the cause of the hypersensitivity reaction. The obliterative condition of airway is reversible and is caused by combinational factors of bronchial smooth muscle spasm, tracheal mucosa edema, increase in the mucus secretion and cellular infiltration to airway wall ,etc. Before, the bronchus contraction had been defined as a main factor, now, it is suggested that inflammation of airway is important. Actually, inflammation with infiltration of not only eosinophilic leukocytes and lymphocytes, but also one of neutrophils and mast cells is seen. The number of eosinophilic leukocytes in the secretion of airway well relates to the degree of a serious illness of the asthma. Inflammatory mediator in the airway secretion of asthmatic patients such as histamine and leukotrienes is released or formed by the result of the allergic reaction in the lung. Cytokines produced from leukocytes and helper T cells, for example, IL-4, IL-5, etc. promote or activate the growth and the differentiation of inflammatory cells, and lead the cells to migration into airway, and prolong the survival of the cells in the place, consequently, inflammation of airway continue.

β-Adrenergic receptor agonists and bronchodilators such as theophylline are used extensively in a spasm of the asthma. Anticholinergic medicines, effects of which are just little, interrupt cholinergic course that causes the obliteration of airway. Corticosteroids that ease the symptom of asthma are used for a pharmacotherapy for a long time. Expectorants are used together for asthmatic patients for whom the spitting of viscosity phlegm is difficult.

However, at present, only expectorants as a symptomatic treatment are used for the grave asthmatic for whom the airway mucus secretion is excess. If the airway mucus secretion could be controlled fundamentally, the possibility to ease the symptoms of critically ill patients remarkably and or to improve is high, and the supply of the medicine that has such action is wished earnestly.

It was reported that a compound (JTP-3913) with the inhibitory activity against elastase and the suppressive activity of neutrophil or eosinophilic leukocyte infiltration could improve the symptoms of asthma through significant repression of neutrophil infiltration and repression of the inflammation cell infiltration in hamster asthmatic model [43rd the general meeting summary of japanese society of allergology, No.290(1993)]. However, this knowledge doesn't suggest how the inhibitory activity against elastase influences the airway mucus secretion.

Moreover, it was reported that a compound (ONO-5046) with the inhibitory activity against elastase could suppress the augmentative airway mucus secretion in a inflammation model of airway by the ozone exposure [Eur. J. Pharmacology, 390(1-2), 197-202 (2000)], which is a model that has similar lesion to the chronic bronchitis, but not a asthmatic model.

For treatment of diseases which the airway mucus secretion unusually deteriorates, such as treatment of asthma, the supply of restrainers of airway mucus secretion is wished earnestly, still it isn't achieved. It is expecting that a part of compounds with the inhibitory activity against elastase are adapted for the chronic bronchitis with asthma and the augmentative airway mucus secretion, all compounds with the inhibitory activity against elastase aren't effective in them. It is suggested that the compound could only be adapted for treatment of the asthma, etc. with the augmentative airway mucus secretion when it is proved that certain compound regulates the airway mucus secretion by the experiment.

### DISCLOSURE OF THE INVENTION

As a result that the present inventors examined assiduously under the above conditions,

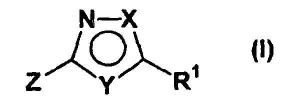

it was first found that serine protease inhibitors, especially five-member heterocyclic compounds represented by the formula (I) (all signs in the formula show the same meanings as postscripts.), with inhibitory activity against elastase, or these nontoxic salts are effective in the repression of the airway mucus secretion, and the present invention was completed.

That is, the present invention relates to, 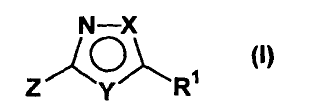 (wherein Z represents the group to contain α-aminocarbonyl group that the carbonyl carbon atom combines with carbon atom of heterocyclic group through covalent bond,
R¹ represents (1) alkyl, alkenyl or alkynyl groups, with the proviso that these groups may be substituted with one or more groups chosen from (a)halogen atom, (b)hydroxy, (c)cyano, (d)nitro, (e)haloalkyl, (f)alkylamino, (g)dialkylamino (h)alkoxy, (l)haloalkoxy, (j)carboxy, (k)carboalkoxy, (l)alkylcaroxamide, (m)arylcaroxamide or (n)-O-(C5-C6) aryl groups,
(2)hydroxy,
(3)amino,
(4)alkylamino,
(5)dialkylamino, or
(6)cycloalkyl, alkylcycloalkyl, alkenylcycloalkyl, cycloalkenyl, alkylcycloalkenyl, alkenylcycloalkenyl, (C5-C12)aryl, (C5-C12)arylalkyl, (C5-C12)arylalkenyl, condensable (C5-C12)aryl-cycloalkyl or alkyl condensable (C5-C12)aryl-cycloalkyl, with the proviso that these groups may be substituted with groups chosen from (a)halogen atom, (b)hydroxy, (c)cyano, (d)nitro, (e)haloalkyl, (f)amino, (g)aminoalkyl, (h)dialkylamino, (i)alkyl, (j)alkenyl, (k)alkylenedioxy, (l)alkynyl, (m)alkoxy, (n)haloalkoxy, (o)carboxy, (p)carboalkoxy, (q)carboxamide, (r)(C5-C6)aryl, (s)-O-(C5-C6)aryl, (t)arylcarboxamide, (u)alkylthio or (v)haloaklylthio that may include 1-4 of hetero atoms chosen from nitrogen, oxygen or sulfur atom, respectively,
X and Y each independently represent oxygen, sulfur, nitrogen or atom, which nitrogen atom may be substituted with groups chosen from
(1)alkyl or alkenyl that may be substituted with 1-3 of halogen atom,
(2)alkynyl,
(3)(C5-C6)aryl, arylalkyl, arylalkenyl that may include 1-3 of hetero atoms chosen from nitrogen, oxygen or sulfur atom or may be substituted with groups chosen from (a)halogen atom, (b)cyano, (c)nitro, (b)hydroxy, (e)haloalkyl, (f)amino, (g)aminoalkyl, (h)dialkylamino, (i)alkyl, (j)alkenyl, (k)alkynyl, (l)alkoxy, (m)haloalkoxy, (n)carboxy, (o)carboalkoxy, (p)carboxamide, (q)arylcarboxamide, (r)alkylthio or (s)haloaklylthio);
prophylactic drugs and/or medicines that include five-member heterocyclic compounds represented by formula (I) or these nontoxic salts as active principle for diseases, with the augmentative airway mucus secretion.

These compounds represented by formula (I) are mentioned in WO96/16080 and WO98/24806 and are mentioned that they could be used for therapy and/or prophylaxis of adult respiratory distress syndrome, septicemic shock, multiple organopathy, myocardial ischemia, reperfusion injury, emphysema, arthritis, periodontal disease, nephritis, dermatitis, psoriasis, cystic fibrosis, chronic bronchitis, atherosclerosis, Alzheimer's disease, organ transplant, corneal ulcer and invasion of malignant tumor. However, there is no mention related to asthma and restrainers of the airway mucus secretion in the above specifications, of course, the validity isn't confirmed. If they don't try with the pathological model of the applicable disease, it doesn't know whether compounds are effective in the sickness.

Z in compounds shown in the formula (I) used for the present invention represents the group containing carbonyl group.

In compounds represented by the formula (I), compounds of which Z is represented by some of the following groups chosen from the formula (I-1) to (I-4), or these non-toxic salts are desirable.

More particularly, in compounds represented by the formula (I), the compound represented by the formula (I) of which Z is represented
by the formula (I-1), 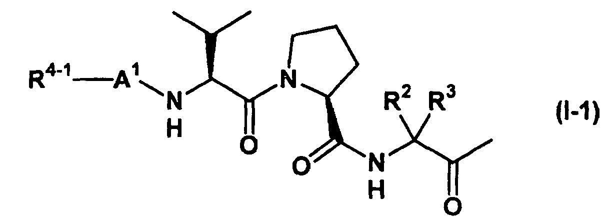 (wherein R² and R³ each independently represent alkyl, alkenyl, -RCOR', -RCOOR', -RNR'R"R⁰, or -RC(O)NR'R"[wherein R represents alkyl or alkenyl, R', R" and R⁰ each independently represent hydrogen atom, alkyl, alkenyl, cycloalkyl or (C5-C6)aryl.] with the proviso that the above groups optionally be substituted with hydrogen, 1-3 of halogen atom, hydroxy, sulfur atom, alkylthio, amino, alkylamino, dialkylamino, alkylguanidinyl, dialkylguanidinyl, guanidinyl or amidylguanidine; or cycloalkyl, alkylcycloalkyl, alkenylcycloalkyl, alkyl-oxyaryl, alkyl-thioaryl, alkyl-aminoaryl, (C5-C12)aryl, (C5-C12)arylalkyl or (C5-C12)arylalkenyl with the proviso that the above groups optionally include 1-4 of hetero atoms chosen from nitrogen, oxygen or sulfur atom and are substituted with halogen atoms, cyano, keto, nitro, hydroxy, haloalkyl, amino, aminoalkyl, dialkylamino, amidine, alkylamidine, dialkylamidine, alkyl, alkenyl, alkylenedioxy, alkynyl, alkoxy, haloalkoxy, carboxy, carboalkoxy, alkylcarboxamide, (C5-C6)aryl, -O-(C5-C6)aryl, arylcarboxamide, alkylthio or haloalkylthio,
A¹ represents direct coupling, -C(O)-, -NH-C(O)-, -S(O)₂-, -NH-S(O)₂-, -C(O)-, -C- or, for example, amino acids chosen from the following, amino acids aren't limited to these;
proline, isoleucine or cyclohexylalanine; cysteine, phenylalanine, homophenylalanine, dehydrophenylalanine, indoline-2-carboxylic acid with the proviso that the above amino acids optionally be substituted with alkyl, alkenyl or phenyl, and that sulfur atom optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthio; tetrahydroisoquinoline-2-carboxylic acid which optionally be substituted in alkyl, alkenyl or phenyl, with the proviso that these groups optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthiotryptophan; tryptophane, tyrosine, serine, threonine; histidine, methionine, valine, norvaline, norleucine or octahydroindole-2-carboxylic acid with the proviso that these amino acids optionally be substituted with alkyl or aryl; asparagine, glutamine, ornithine or lysine with the proviso that nitrogen atoms in side chains of these amino acids optionally be substituted with alkyl, alkenyl, alkynyl, alkoxyalkyl, alkylthioalkyl, alkylaminoalkyl, dialkylaminoalkyl, carboxyalkyl, alkoxyalkyl, alkylthioalkyl, alkylaminoalkyl, dialkylaminoalkyl, carboxyalkyl, alkoxycarbonylalkyl, aryl, arylalkyl, cycloalkyl, alkylcycloalkyl, condensed aryl-cycloalkyl or alkyl condensed aryl-cycloalkyl and optionally include one or more hetero atoms chosen from nitrogen, oxygen or sulfur atom,
R⁴⁻¹ represents cycloalkyl, alkylcycloalkyl, (C5-C12)aryl, (C5-C12)arylalkyl, condensed (C5-12)aryl-cycloalkyl or condensed alkyl(C5-C12)aryl-cycloalkyl with the proviso that these groups optionally include hydrogen atom, alkyl, alkenyl or alkynyl, one or more hetero atoms chosen from nitrogen, oxygen or sulfur atom, and optionally be substituted with alkyl, alkenyl, alkynyl, halogen atom, cyano, nitro, hydroxy, haloalkyl, alkoxy, amino, aminoalkyl, dialkylamino, carboxy, haloalkoxy, carboalkoxy, alkylcarboxamide, aryl, arylalkyl, arylcarboxamide, alkylthio or haloalkylthio);
the formula (1-2), 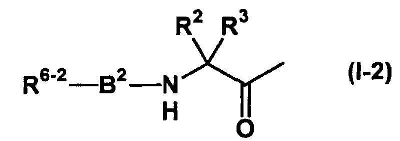 (wherein R² and R³ represent the same meanings as aforesaid;
B² represents -S(O)₂-, -C(O)-, -OC(O)- or -CH₂C(O)-;
R⁶⁻² represents the following groups; 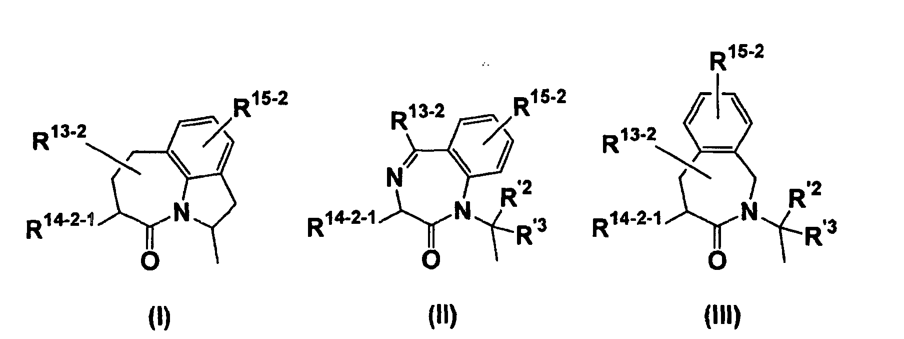 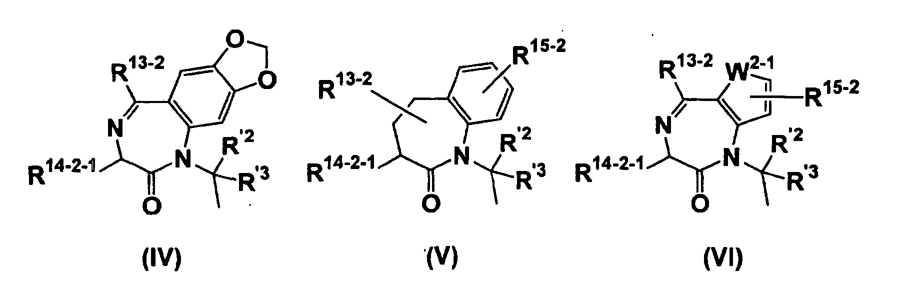 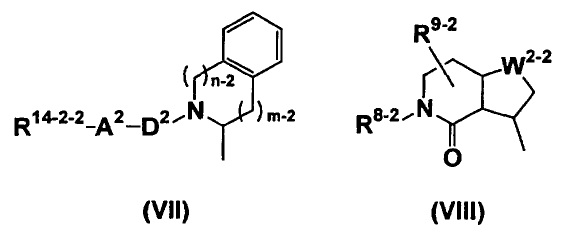 (wherein R'² and R'³ represent the same meanings as R² and R³;
R¹³⁻² represents aryl, arylalkyl, cycloalkyl, alkylcycloalkyl, condensed aryl-cycloalkyl, alkyl condensed aryl-cycloalkyl with the proviso that these groups optionally include hydrogen atom, alkyl, halogen atom, alkoxy, carboalkoxy, carboxy, alkylthio, amino alkylamino, dialkylamino or one or more hetero atoms chosen from nitrogen, oxygen or sulfur atom, and optionally be substituted with halogen atom or alkyl;
R¹⁴⁻²⁻¹ represents aryl, arylalkyl, cycloalkyl, alkylcycloalkyl, condensed aryl-cycloalkyl, alkyl condensed aryl-cycloalkyl or aryloxycarboxamide with the proviso that these groups optionally include hydrogen atom, alkyl, alkenyl, amino, alkylamino, dialkylamino or one or more hetero atoms chosen from nitrogen, oxygen or sulfur atom, and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, arylalkylcarboxamide, alkylthio or haloalkylthio;
R¹⁵⁻² represents aryl, arylalkyl, cycloalkyl, alkylcycloalkyl, condensed aryl-cycloalkyl or alkyl condensed aryl-cycloalkyl with the proviso that these groups optionally include halogen atom, alkyl, halogen atom, alkoxy, carboalkoxy, carboxy, alkylthio, amino, alkylamino, dialkylamino or one or more hetero atoms chosen from nitrogen, oxygen or sulfur atom;
W²⁻¹ represents oxygen or sulfur atom; carbon or nitrogen atom that optionally be substituted with hydrogen atom, alkyl or aryl;
[m-2] represents 0 or 1;
[n-2] represents 0 or 1;
D² represents direct coupling or, for example, amino acids chosen from the following, amino acids aren't limited to these;
proline, isoleucine or cyclohexylalanine; cysteine, phenylalanine, homophenylalanine, dehydrophenylalanine, indoline-2-carboxylic acid with the proviso that the above amino acids optionally be substituted with alkyl, alkenyl or phenyl, and that sulfur atom optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthio; tetrahydroisoquinoline-2-carboxylic acid which optionally be substituted in alkyl, alkenyl or phenyl, with the proviso that these groups optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthiotryptophan; tryptophane, tyrosine, serine, threonine; histidine, methionine, valine, norvaline, norleucine or octahydroindole-2-carboxylic acid with the proviso that these amino acids optionally be substituted with alkyl or aryl; asparagine, glutamine, ornithine or lysine with the proviso that nitrogen atoms in side chains of these amino acids optionally be substituted with alkyl, alkenyl, alkynyl, alkoxyalkyl, alkylthioalkyl, alkylaminoalkyl, dialkylaminoalkyl, carboxyalkyl, alkoxyalkyl, alkylthioalkyl, alkylaminoalkyl, dialkylaminoalkyl, carboxyalkyl, alkoxycarbonylalkyl, aryl, arylalkyl, cycloalkyl, alkylcycloalkyl, condensed aryl-cycloalkyl or alkyl condensed aryl-cycloalkyl and optionally include one or more hetero atoms chosen from nitrogen, oxygen or sulfur atom;
A² represents direct coupling, -C(O)-, -NH-C(O)-, -S(O)₂-, -NH-S(O)₂-, -C(O)- or -C-;
R¹⁴⁻²⁻² represents aryl, arylalkyl, cycloalkyl, alkylcycloalkyl, condensed aryl-cycloalkyl or alkyl condensed aryl-cycloalkyl with the proviso that these groups optionally include hydrogen atom, alkyl, alkenyl, amino, alkylamino, dialkylamino or one or more hetero atoms chosen from nitrogen, oxygen or sulfur atom, and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, alkylthio or haloalkylthio;
W²⁻² represents sulfur or oxygen atom;
R⁸⁻² represents alkylamino dialkylamino or amino;
R⁹⁻² represents hydrogen atom, alkyl or halogen atom)); the formula (I-3). 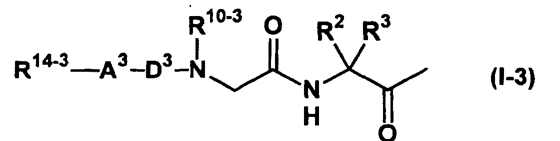 (wherein R² and R³ represent the same meanings as aforesaid,
R¹⁰⁻³ represents (C5-C6)aryl, (C5-C6)arylalkyl, (C5-C6)arylalkenyl, cycloalkyl, condensed aryl-cycloalkyl with the proviso that these groups optionally include one or more hetero atoms chosen from nitrogen, sulfur or non-peroxideone oxygen atom, and optionally be substituted with halogen atom, cyano, nitro, haloalkyl, amino, aminoalkyl, dialkylamino, alkyl, alkenyl, alkoxy, haloalkoxy, carboxy, carboalkoxy, alkylcarboxamide, alkylthio or haloalkylthio;
D³ represents direct coupling, -C(O)- or, for example, amino acids chosen from the following, amino acids aren't limited to these;
proline, isoleucine or cyclohexylalanine; cysteine, phenylalanine, homophenylalanine, dehydrophenylalanine, indoline-2-carboxylic acid with the proviso that the above amino acids optionally be substituted with alkyl, alkenyl or phenyl, and that sulfur atom optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthio; tetrahydroisoquinoline-2-carboxylic acid which optionally be substituted in alkyl, alkenyl or phenyl, with the proviso that these groups optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthiotryptophan; tryptophane, tyrosine, serine, threonine; histidine, methionine, valine, norvaline, norleucine or octahydroindole-2-carboxylic acid with the proviso that these amino acids optionally be substituted with alkyl or aryl; asparagine, glutamine, ornithine or lysine with the proviso that nitrogen atoms in side chains of these amino acids optionally be substituted with alkyl, alkenyl, alkynyl, alkoxyalkyl, alkylthioalkyl, alkylaminoalkyl, dialkylaminoalkyl, carboxyalkyl, alkoxyalkyl, alkylthioalkyl, alkylaminoalkyl, dialkylaminoalkyl, carboxyalkyl, alkoxycarbonylalkyl, aryl, arylalkyl, cycloalkyl, alkylcycloalkyl, condensed aryl-cycloalkyl or alkyl condensed aryl-cycloalkyl and optionally include one or more hetero atoms chosen from nitrogen, oxygen or sulfur atom;
A³ represents direct coupling, -C(O)-, -NH-C(O)-, -S(O)₂-, -NH-S(O)₂-, -C(O)-, -S(O)2-NH-, OC(0)NH-, -OC(O)- or -C-;
R¹⁴⁻² and R¹⁴⁻²⁻¹ represent the same meanings as aforesaid),
the formula (I-4); 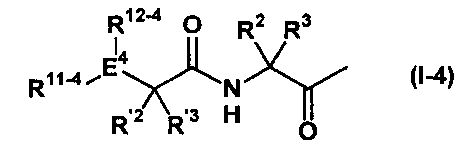 (wherein R², R³ R'² and R'³ represent the same meanings as aforesaid,
R¹¹⁻⁴, R¹²⁻⁴ and E⁴ are constituted of monocyclic or dicyclic ring with the proviso that these rings include 5-10 atoms chosen from carbon, nitrogen, sulfur or oxygen atom and include one or more keto groups and optionally be substituted with halogen atom, cyano, nitro, haloalkyl, amino, aminoalkyl, dialkylamino, alkyl, alkenyl, alkynyl, alkoxy, haloalkoxy, carboxy, carboalkoxy, alkylcarboxamide, alkylthio or haloalkylthio; and represent cycloalkyl, alkylcycloalkyl, alkenylcycloalkyl, (C5-C12)aryl, (C5-C12)arylalkyl, ((C5-C12)arylalkyl)OC(O)NH- or (C5-C12)arylalkyl with the proviso that these groups optionally include one or more hetero atoms chosen from nitrogen, sulfur or non-peroxideone oxygen atom, and optionally be substituted with halogen atom, cyano, nitro, haloalkyl, amino, aminoalkyl, dialkylamino, alkyl, alkenyl, alkoxy, haloalkoxy, carboxy, carboalkoxy, -C(O)O(alkyl), -C(O)(alkyl), alkylcarboxamide, alkylthio or haloalkylthio) or these nontoxic salts are desirable.

Further, in the above formula (I-4), R¹¹⁻⁴, R¹²⁻⁴ and E⁴ are constituted of a ring, respectively. Concretely, for R¹¹⁻⁴, R¹²⁻⁴ and E⁴, groups chosen from the following formula (I-4-1) to formula (I-4-13) are given.

If Z in compounds represented by the formula (I) of the present invention is a group represented by the formula (I-4), these compounds that R¹¹⁻⁴, R¹²⁻⁴ and E⁴ are represented by groups chosen from the following formula (I-4-1) to formula (I-4-13) or these nontoxic salts are desirable.

More particularly, if Z in compounds represented by the formula (I) of the present invention is a group represented by the formula (I-4), compounds represented by the formula (I) that R¹¹⁻⁴, R¹²⁻⁴ and E⁴ are represented
by the formula (I-4-1), 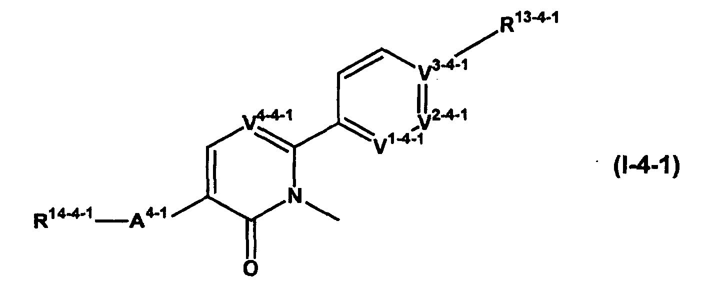 (wherein A⁴⁻¹ represents the same meanings as A³,
V¹⁻⁴⁻¹, V²⁻⁴⁻¹, V³⁻⁴⁻¹ and V⁴⁻⁴⁻¹ each independently represent carbon or nitrogen atom, in case V³⁻⁴⁻¹ represents carbon atom, R¹³⁻⁴⁻¹ represents hydrogen atom, alkyl, halogen atom, alkoxy, carboalkoxy, carboxy, alkylthio, amino, alkylamino, dialkylamino; aryl, arylalkyl, cycloalkyl, alkylcycloalkyl, condensed aryl-cycloalkyl or alkyl condensed aryl-cycloalkyl with the proviso that these groups optionally include one or more hetero atoms chosen from nitrogen, sulfur or oxygen atom, and optionally be substituted with halogen atom or alkyl;
R¹⁴⁻⁴⁻¹ represents hydrogen atom, alkyl, alkenyl, amino, alkylamino or dialkylamino; aryl, arylalkyl, cycloalkyl, alkylcycloalkyl, condensed aryl-cycloalkyl or alkyl condensed aryl-cycloalkyl, arylalkylcarbonyl or arylalkylcarboxamiae with the proviso that these groups optionally include one or more hetero atoms chosen from nitrogen, sulfur or oxygen atom, and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, alkylthio or haloalkylthio),
the formula (I-4-2), 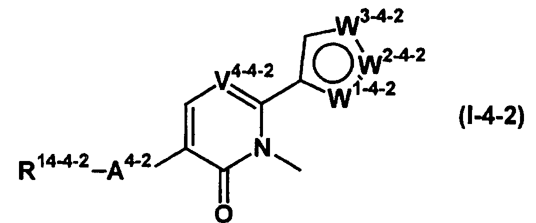 (wherein R¹⁴⁻⁴⁻² represents the same meanings as R¹⁴⁻⁴⁻¹,
A⁴⁻² represents the same meanings as A⁴⁻¹,
V⁴⁻⁴⁻² represents the same meanings as A⁴⁻⁴⁻¹,
W¹⁻⁴⁻², W²⁻⁴⁻² and W³⁻⁴⁻² each independently represent nitrogen, carbon or oxygen atom that optionally be substituted with alkyl),
the formula (I-4-3), 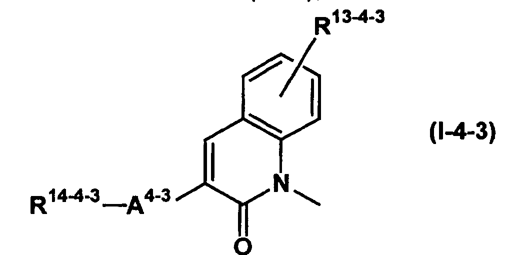 (wherein R¹⁴⁻⁴⁻³ represents the same meanings as R¹⁴⁻⁴⁻¹,
A⁴⁻³ represents the same meanings as A⁴⁻¹,
R¹³⁻⁴⁻³ represents the same meanings as R¹³⁻⁴⁻¹),
the formula (I-4-4), 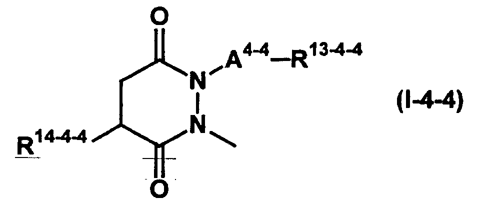 (wherein A⁴⁻⁴ represents direct coupling, -C- or -C(O)-,
R¹³⁻⁴⁻⁴ represents the same meanings as R¹³⁻⁴⁻¹,
R¹⁴⁻⁴⁻⁴ represents the same meanings as R¹⁴⁻⁴⁻¹),
the formula (I-4-5), 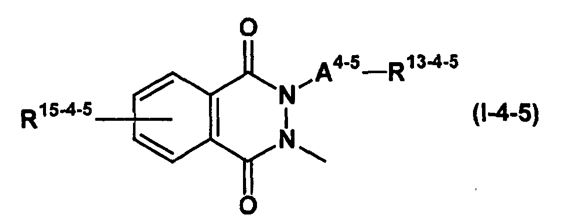 (wherein A⁴⁻⁵ represents the same meanings as A⁴⁻⁴,
R¹³⁻⁴⁻⁵ represents the same meanings as R¹³⁻⁴⁻¹,
R¹⁵⁻⁴⁻⁵ represents the same meanings as R¹⁵⁻²),
the formula (I-4-6). 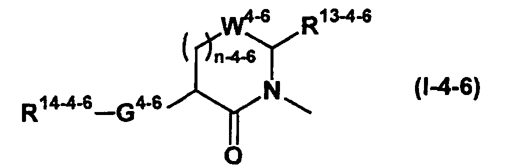 (wherein W⁴⁻⁵ represents sulfur atom, SO, SO₂, or carbon atom,
[n-4-6] represents zero, one or two,
R13-4-6 represents the same meanings as R¹³⁻⁴⁻¹,
R¹⁴⁻⁴⁻⁶ represents the same meanings as R¹⁴⁻⁴⁻¹,
G⁴⁻⁶ represents -NHC(O)-, -OC(O)NH-, -C(O)-, -NHS(O)₂- or direct coupling),
the formula (I-4-7), 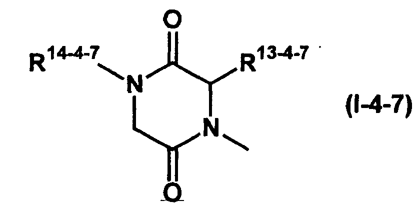 (wherein R¹³⁻⁴⁻⁷ represents the same meanings as R¹³⁻⁴⁻¹, or represents CH=R¹⁵⁻⁴⁻⁷ or R¹⁵⁻⁴⁻⁷,
R¹⁵⁻⁴⁻⁷ represents pyridinyl, phenyl or benzyl that optionally be substituted with halogen atom, dialkylamino or -C(O)OCH₂,
R¹⁴⁻⁴⁻⁷ represents hydrogen atom, alkyl, alkenyl, CH₂C(O)-; aryl, arylalkyl, cycloalkyl, alkylcycloalkyl, condensed aryl-cycloalkyl or alkyl condensed aryl-cycloalkyl, aryloxycarbonyl or arylalkyloxycarbonyl with the proviso that these groups optionally include one or more hetero atoms chosen from nitrogen, sulfur or oxygen atom, and optionally be substituted with alkyl, halogen atom, alkoxy, amino, alkylamino, dialkylamino, carboxy, alkenyl, haloalkoxy, carboalkoxy, alkylcarboxamide, aryl, arylalkyl, arylcarboxamide, alkylthio or haloalkylthio),
the formula (I-4-8), 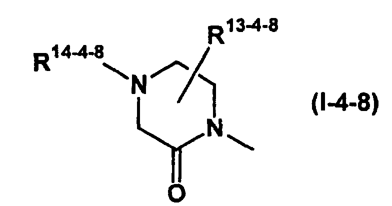 (wherein R¹³⁻⁴⁻⁸ represents the same meanings as R¹³⁻⁴⁻⁷,
R¹⁴⁻⁴⁻⁸ represents the same meanings as R¹⁴⁻⁴⁻⁷),
the formula (I-4-9), 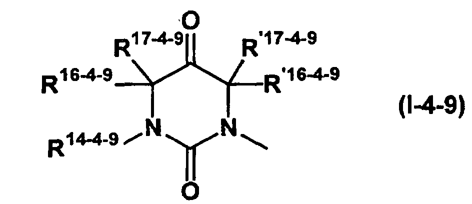 (wherein R¹⁴⁻⁴⁻⁹ represents the same meanings as R¹⁴⁻⁴⁻⁷,
R¹⁶⁻⁴⁻⁹, R¹⁷⁻⁴⁻⁹, R'¹⁶⁻⁴⁻⁹ and R'¹⁷⁻⁴⁻⁹ each independently represent hydrogen atom, alkyl, alkenyl, alkylthio, alkylthioalkyl or guanidine; cycloalkyl, cycloalkeny, alkylcycloalkyl, aryl, arylalkyl or arylalkenyl that optionally be substituted with carboalkoxy, hydroxy, haloalkyl, alkylthio, alkylguanidine, dialkyl guanidine or amidine),
the formula (I-4-10), 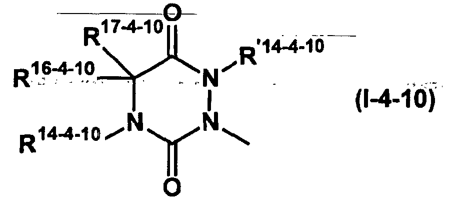 (wherein R¹⁴⁻⁴⁻¹⁰ and R'¹⁴⁻⁴⁻¹⁰ each independently represent the same meanings as R¹⁴⁻⁴⁻⁷,
R¹⁵⁻⁴⁻¹⁰ represents the same meanings as R¹⁵⁻⁴⁻⁹,
R¹⁷⁻⁴⁻¹⁰ represents the same meanings as R¹⁷⁻⁴⁻⁹),
the formula (I-4-11). 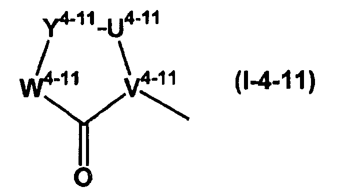 [wherein U⁴⁻¹¹, V⁴⁻¹¹, W⁴⁻¹¹ and Y⁴⁻¹¹ each independently represent nitrogen, carbon atom, C(O), N(R¹³⁻⁴⁻¹¹)(wherein R¹²⁻⁴⁻¹¹ represents hydrogen atom, alkyl, halogen atom, alkoxy, carbalkoxy, carboxy, alkylthio, amino, alkylamino, dialkylamino; aryl, arylalkyl, cycloalkyl, alkylcycloalkyl, condensed aryl-cycloalkyl or alkyl condensed aryl-cycloalkyl with the proviso that these groups optionally include one or more hetero atoms chosen from nitrogen, sulfur or oxygen atom and optionally be substituted with halogen atom or alkyl.), N(R¹⁴⁻⁴⁻¹¹)(wherein R¹⁴⁻⁴⁻¹¹ represents hydrogen atom, alkyl, alkenyl; aryl, arylalkyl, cycloalkyl, alkylcycloalkyl, condensed aryl-cycloalkyl or alkyl condensed aryl-cycloalkyl with the proviso that these groups optionally include one or more hetero atoms chosen from nitrogen, sulfur or oxygen atom and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, alkylthio or haloalkylthio.) or C(R¹⁵⁻⁴⁻¹¹)(R¹⁷⁻⁴⁻¹¹) (wherein R¹⁵⁻⁴⁻¹¹ and R¹⁷⁻⁴⁻¹¹ each independently are constituted of hydrogen atom, alkyl, alkylthio, alkylthioalkyl, carboxylate represented by the for formula -(CH₂)ₘ₋₄₋₁₁C(O)OR⁰⁻⁴⁻¹¹ or N-substituted alkylamide presented by -(CH₂)ₘ₋₄₋₁₁C(O)NR⁰⁻⁴⁻¹¹R'⁰⁻⁴⁻¹¹(wherein [m-4-11] represents an integer of 1 to 6, and R0-4-11 and R'0-4-11 each independently represent aryl, arylalkyl, cycloalkyl, alkylcycloalkyl, condensed aryl-cycloalkyl or alkyl condensed aryl-cycloalkyl with the proviso that these groups optionally include one or more hetero atoms chosen from nitrogen, sulfur or non-peroxideone oxygen atom and optionally be substituted with amino, alkylamino, dialkylamino, guanidine, carboalkoxy, keto, hydroxy, alkyl, haloalkyl, alkylthio, alkylguanidine, dialkylguanidine or amidine.) or rings that include 4-8 atoms chosen from carbon, nitrogen, oxygen atom or sulfur atom.)],
the formula (I-4-12), 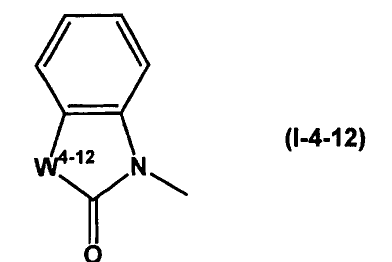 (wherein W⁴⁻¹² represents the same meanings as W⁴⁻¹¹),
the formula (I-4-13), 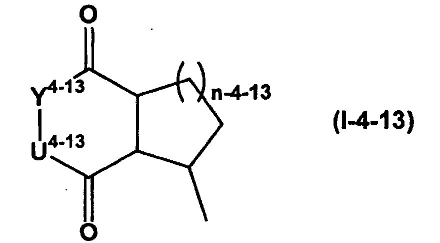 [wherein U⁴⁻¹² and V⁴⁻¹² each independently represent nitrogen, carbon atom, N(R¹³⁻⁴⁻¹³) or C(R¹⁶⁻⁴⁻¹³)(R¹⁷⁻⁴⁻¹³)(wherein R¹³⁻⁴⁻¹³ represents hydrogen atom, alkyl, alkoxy, carbalkoxy, carboxy, alkylthio, amino, alkylamino, dialkylamino; aryl, arylalkyl, cycloalkyl, alkylcycloalkyl, condensed aryl-cycloalkyl or alkyl condensed aryl-cycloalkyl with the proviso that these groups optionally include one or more hetero atoms chosen from oxygen, nitrogen or sulfur atom, and R¹⁶⁻⁴⁻¹³ represents the same meanings as R¹⁶⁻⁴⁻¹¹,
R¹⁷⁻⁴⁻¹³ represents the same meanings as R¹⁶⁻⁴⁻¹¹),
[n-4-13] represents 1 or 2.] or these nontoxic salts are desirable.

In compounds represented by the formula (I) of the present invention, compounds that have been specified concretely as actual examples or examples in specifications of WO96/16080 and WO98/24806 are given as desirable compounds.

As more desirable compounds of them, compounds from the following (1) to (58) or these nontoxic salts are given.
Compound (1): 2-[5-amino-6-oxo-2-(4-fluorophenyl)-1,6-dihydro-1-pyrimidinyl]-N-[(1S)-1-[[5-(3-methylbenzyl)-1,3,4-oxadiazole-2-yl]carbonyl]-2-methylpropyl]acetamide (CAS Registry No. 208840-39-5) 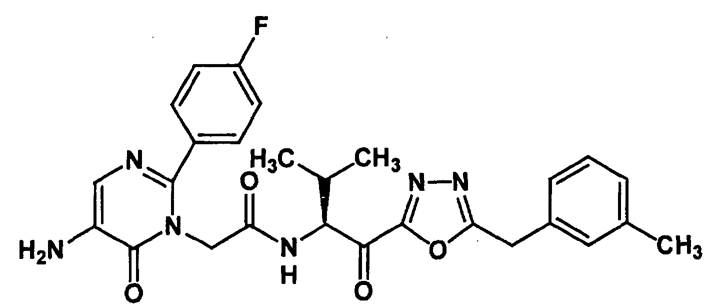
Compound (2): (2S,5S)-4-oxo-amino-5-1,2,4,5,6,7-hexahydro-N-[(1S)-1-[[5-3-methylbenzyl)-1,3,4-oxadiazole-2-yl]carbonyl]-2-methylpropyl]azepino[3,2,1]indole-2-carboxamide (CAS Registry No. 208845-71-0), 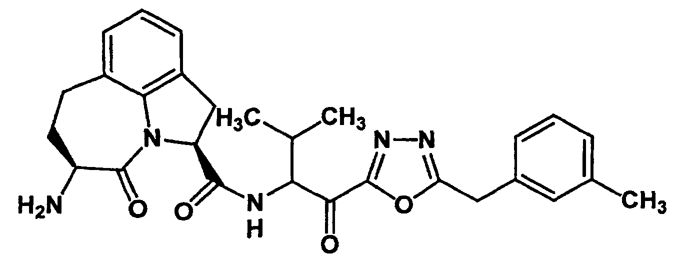
Compound (3): 2-[3[amino-2-oxo-5-phenyl-1,4-benzodiazepinyl]-N-1-[[5-(3-methylbenzyl)-1,3,4-oxadiazole-2-yl]carbonyl]-2-methylpropyl]acetamide (CAS Registry No. 208845-74-3) 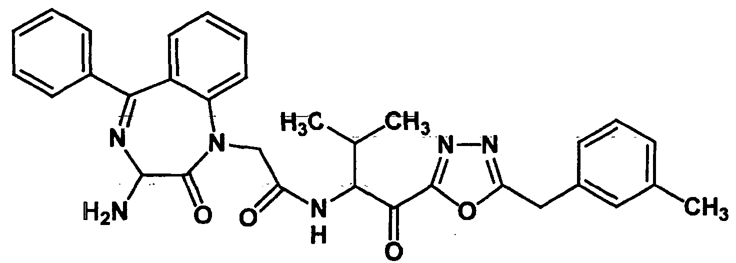
Compound (4): Methylsulfonyl-L-valyl-N-[(1S)-1-[[5-(3-methylbenzyl)-1,3,4-oxadiazole-2-yl]carbonyl]-2-methylpropyl]-L-prolinamide (CAS Registry No. 208846-01-9), 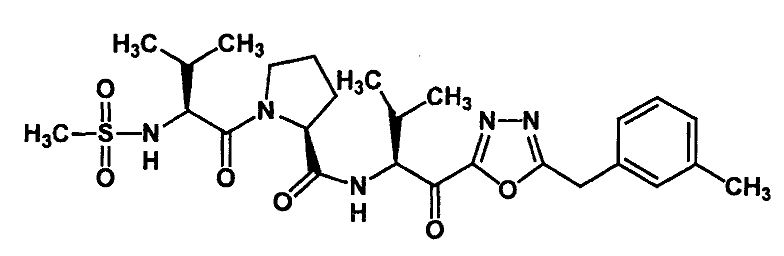
Compound (5): 2-[4-(R)-isopropyl-2,5-imidazolidinedione-1-yl]-N-[(1S)-1-[[5-(3-methylbenzyl)-1,3,4-oxadiazole-2-yl]carbonyl]-2-methylpropyl]acetamide (CAS Registry No. 208846-10-0). 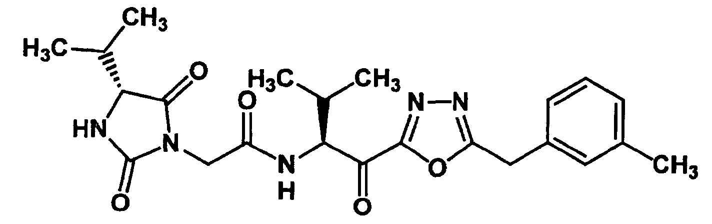
Compound (6): Benzyloxycarbonyl-L-valyl-N-[(1S)-1-[[5-(3-methylbenzyl)-1,3,4-oxadiazole-2-yl]carbonyl]-2-methylpropyl]-L-prolinamide (CAS Registry No. 208846-12-2). 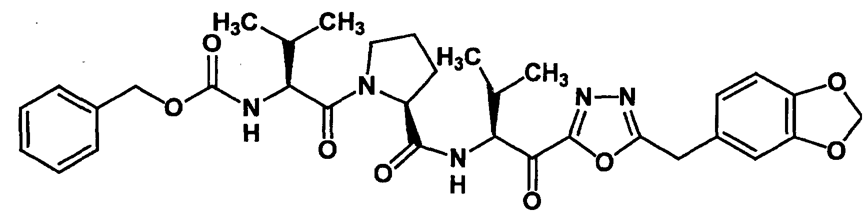
Compound (7): 2-[3-(2-(morpholine-2-yl)ethyl)-4-phenyl-2,5-imidazolidinedione-1-yl]-N-[1 -[[5-(3-methylbenzyl)-1,3,4-oxadiazole-2-yl]carbonyl]-2-methylpropyl] acetamide (CAS Registry No. 208846-26-8), 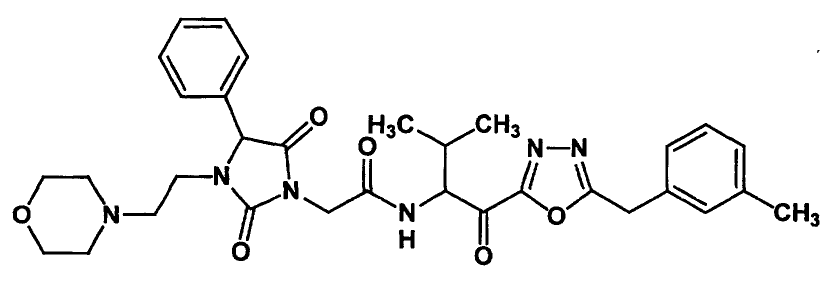
Compound (8): 2-[4-methyl-4-(pyridine-2-yl)-2,5-imidazolidinedione-1-yl]-N-1-[[5-(3-methylbenzyl)-1,3,4-oxadiazole-2-yl]carbonyl]-2-methylpropyl] acetamide (CAS Registry No. 208846-58-6), 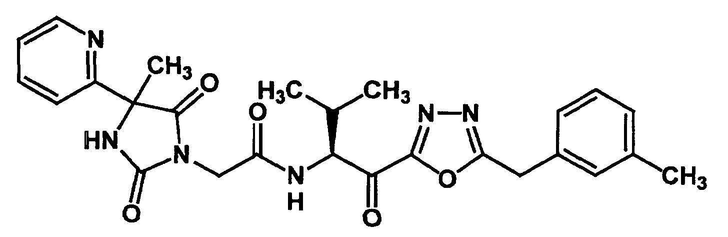
Compound (9): 2-[(4S)-4-(2-methylpropyl)-2,5-imidazolidinedione-1-yl]-N-1-[[5-(3-methylbenzyl)-1,3,4-oxadiazole-2-yl]carbonyl]-2-methylpropyl]acetamide (CAS Registry No. 208846-86-0), 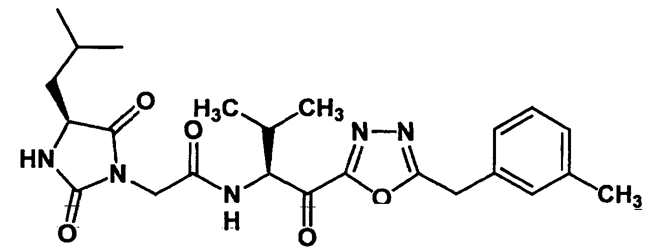
Compound (10): 2-[5-amino-6-oxo-2-(4-fluorophenyl)-1,6-dihydro-1-pyrimidinyl]-N-[(1S)-1-[[5-(α,α-methylbenzyl)-1,3,4-oxadiazole-2-yl]carbonyl]-2-methylpropyl]acetamide (CAS Registry No. 208840-37-3), 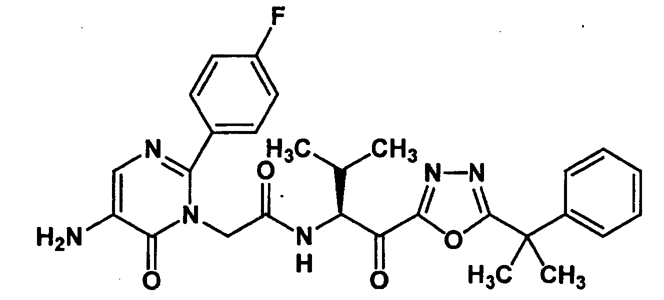
Compound (11): 2-[5-amino-6-oxo-2-(4-fluorophenyl)-1,6-dihydro-1-pyrimidinyl]-N-[(1S)-1-[[5-(α,α-dimethyl-3-methylbenzyl)-1,3,4-oxadiazole-2-yl]carbonyl]-2-methylpropyl]acetamide (CAS Registry No. 208840-38-4), 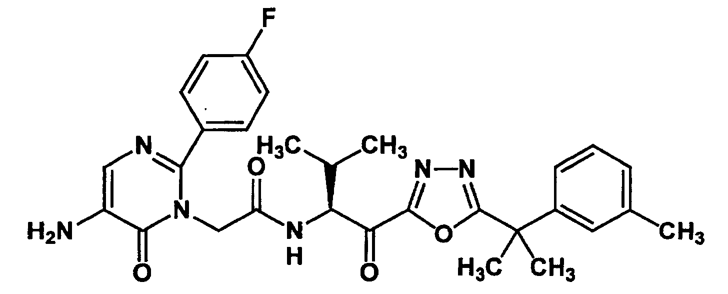
Compound (12): 2-[5-amino-6-oxo-2-(4-fluorophenyl)-1,6-dihydro-1-pyrimidinyl]-N-[(1R)-1-[[5-(3-methylbenzyl)-1,3,4-oxadiazole-2-yl]carbonyl]-2-methylpropyl]acetamide (CAS Registry No. 251988-66-6), 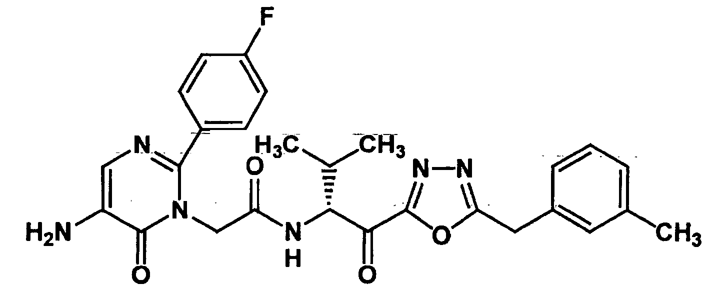
Compound (13): 2-[5-amino-6-oxo-2-(4-fluorophenyl)-1,6-dihydro-1-pyrimidinyl]-N-[(1S)-1-[[5-phenyl-1,3,4-oxadiazole-2-yl]carbonyl]-2-methylpropyl]acetamide (CAS Registry No. 208847-78-3), 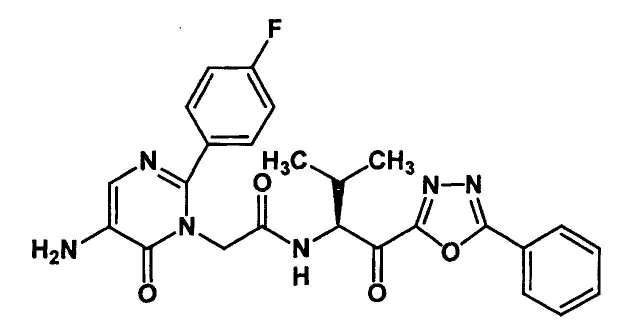
Compound (14): 2-[5-amino-6-oxo-2-(4-fluorophenyl)-1,6-dihydro-1-pyrimidinyl]-N-[(1S)-1-[[5-(t-butyl)-1,3,4-oxadiazole-2-yl]carbonyl]-2-methylpropyl]acetamide (CAS Registry No. 208847-80-7), 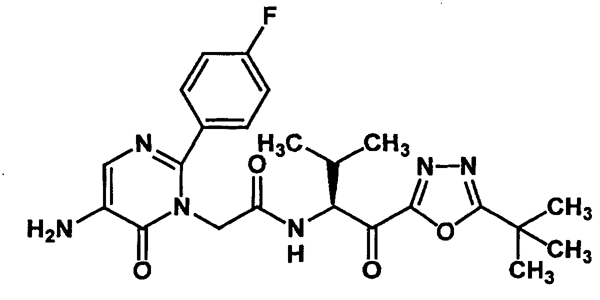
Compound (15): 2-[5-amino-6-oxo-2-(4-fluorophenyl)-1,6-dihydro-1-pyrimidinyl]-N-[(1S)-1-[[5-(α,α-dimethyl-3,4-methylenedioxybenzyl)-1,3,4-oxadiazole-2-yl]carbonyl]-2-methylpropyl]acetamide (CAS Registry No. 208847-84-1), 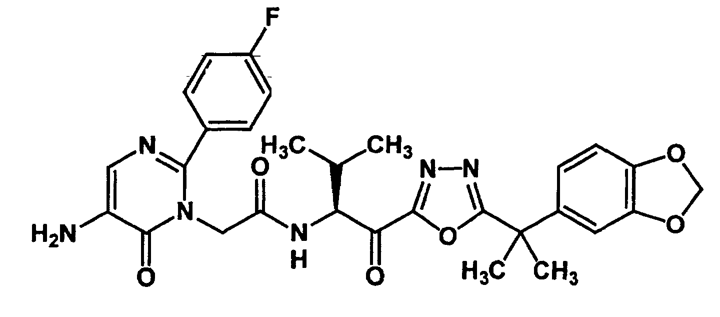
Compound (16): 2-[5-amino-6-oxo-2-(4-fluorophenyl)-1,6-dihydro-1-pyrimidinyl]-N-[(1S)-1-[[5-benzyl-1,3,4-oxadiazole-2-yl]carbonyl]-2-methylpropyl]acetamide (CAS Registry No. 208847-87-4), 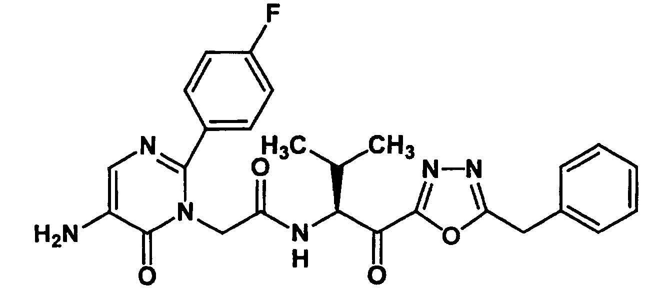
Compound (17): 2-[5-amino-6-oxo-2-(4-fluorophenyl)-1,6-dihydro-1-pyrimidinyl]-N-[(1S)-1-[[5-methyl-1,3,4-oxadiazole-2-yl]carbonyl]-2-methylpropyl]acetamide (CAS Registry No. 208847-88-5), 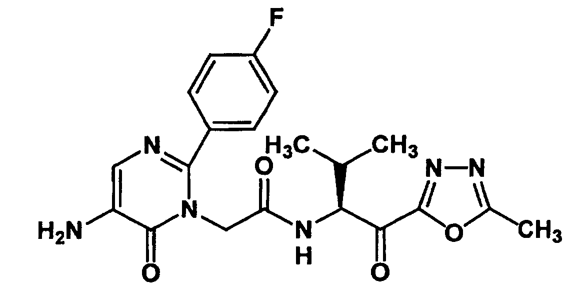
Compound (18): 2-[5-amino-6-oxo-2-(4-fluorophenyl)-1,6-dihydro-1-pyrimidinyl]-N-[(1S)-1-[[5-(1-methylethyl)-1,3,4-oxadiazole-2-yl]carbonyl]-2-methylpropyl]acetamide (CAS Registry No. 208847-89-6), 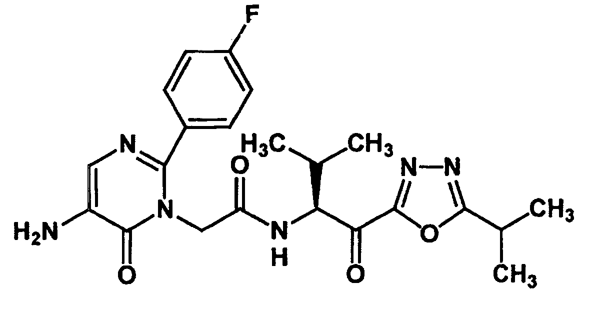
Compound (19): 2-[5-amino-6-oxo-2-(4-fluorophenyl)-1,6-dihydro-1-pyrimidinyl]-N-[(1S)-1-[[5-butyl-1,3,4-oxadiazole-2-yl]carbonyl]-2-methylpropyl]acetamide (CAS Registry No. 208847-90-9), 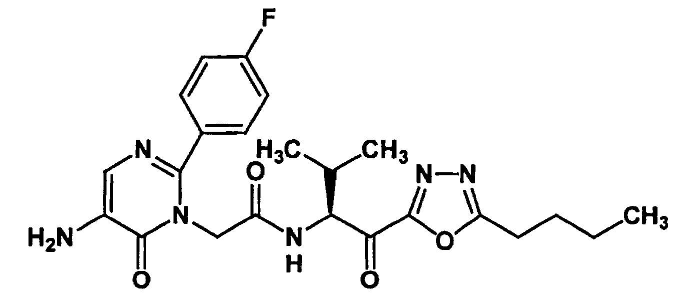
Compound (20): 2-[5-amino-6-oxo-2-phenyl-1,6-dihydro-1-pyrimidinyl]-N-[(1S)-1-[[5-(t-butyl)-1,3,4-oxadiazole-2-yl]carbonyl]-2-methylpropyl]acetamide (CAS Registry No. 208847-91-0), 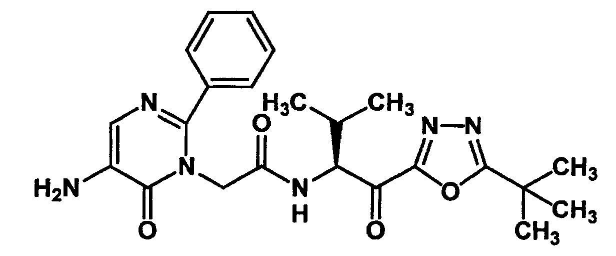
Compound (21): 2-[5-amino-6-oxo-2-phenyl-1,6-dihydro-1-pyrimidinyl]-N-[(1S)-1-[[5-(α,α-dimethylbenzyl)-1,3,4-oxadiazole-2-yl]carbonyl]-2-methylpropyl]acetamide (CAS Registry No. 208847-95-4), 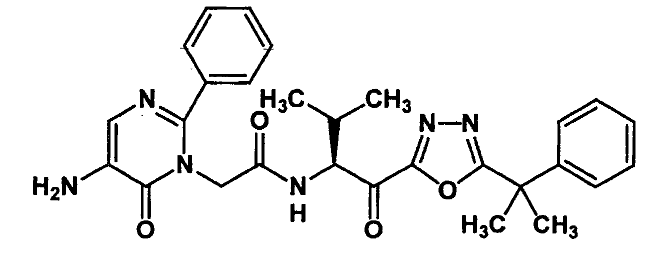
Compound (22): 2-[5-amino-6-oxo-2-(4-fluorophenyl)-1,6-dihydro-1-pyrimidinyl]-N-[(1S)-1-[[5-(1-methylcyclopropyl)-1,3,4-oxadiazole-2-yl]carbonyl]-2-methylpropyl]acetamide (CAS Registry No. 208847-98-7), 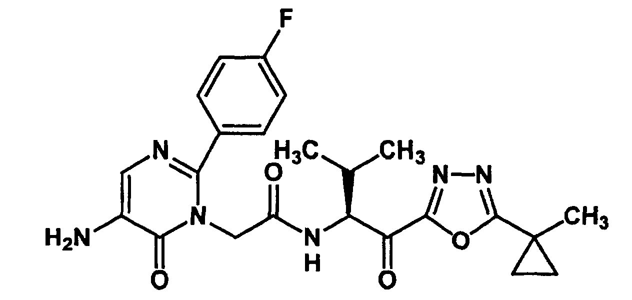
Compound (23): 2-[6-oxo-2-(4-fluorophenyl)-1,6-dihydro-1-pyrimidinyl]-N-[(1S)-1-[[5-(t-butyl)-1,3,4-oxadiazole-2-yl]carbonyl]-2-methylpropyl]acetamide (CAS Registry No. 208848-00-4). 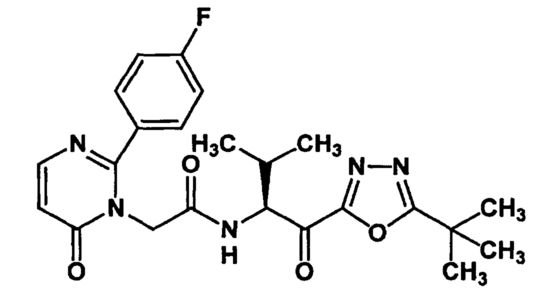
Compound (24): 2-[5-amino-6-oxo-2-(4-fluorophenyl)-1,6-dihydro-1-pyrimidinyl]-N-[(1R)-1-[[5-(t-butyl)-1,3,4-oxadiazole-2-yl]carbonyl]-2-methylpropyl]acetamide (CAS Registry No. 208848-02-6), 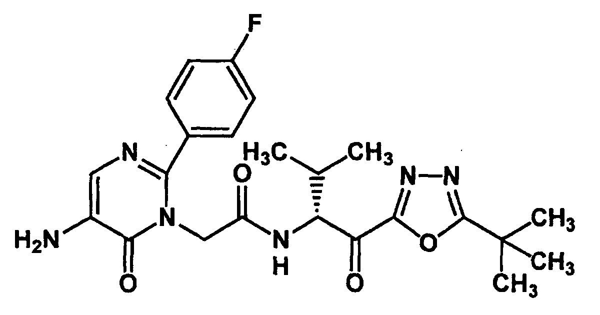
Compound (25): 2-[5-amino-6-oxo-2-(4-fluorophenyl)-1,6-dihydro-1-pyrimidinyl]-N-[(1R)-1 -[[5-(α,α-dimethylbenzyl)-1,3,4-oxadiazole-2-yl]carbonyl]-2-methylpropyl]acetamide (CAS Registry No. 208848-05-9), 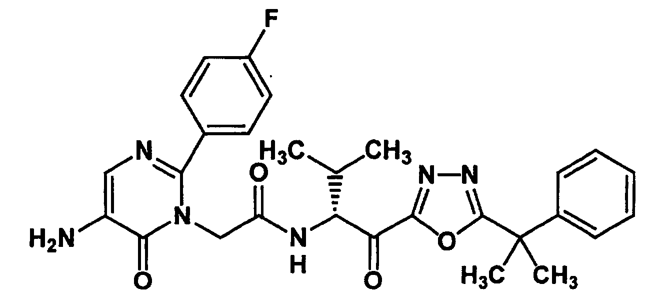
Compound (26): 2-[6-oxo-2-(4-fluorophenyl)-1,6-dihydro-1-pyrimidinyl]-N-[(1S)-1-[[5-(α,α-dimethylbenzyl)-1,3,4-oxadiazole-2-yl]carbonyl]-2-methylpropyl]acetamide (CAS Registry No. 208848-06-0), 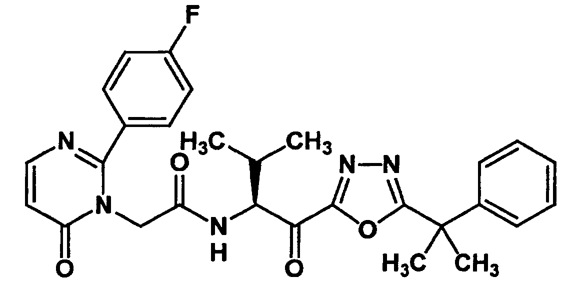
Compound (27): 2-[5-amino-6-oxo-2-phenyl-1,6-dihydro-1-pyrimidinyl]-N-[(1S)-1-[[5-(1-methylcyclopropyl)-1,3,4-oxadiazole-2-yl]carbonyl]-2-methylpropyl]acetamide (CAS Registry No. 208848-12-8), 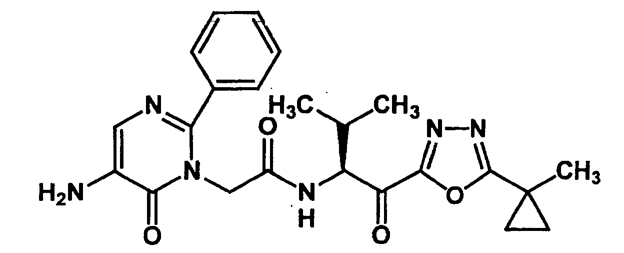
Compound (28): 2-[5-amino-6-oxo-2-phenyl-1,6-dihydro-1-pyrimidinyl]-N-[(1R)-1-[[5-(t-butyl)-1,3,4-oxadiazole-2-yl]carbonyl]-2-methylpropyl]acetamide (CAS Registry No. 208848-13-9), 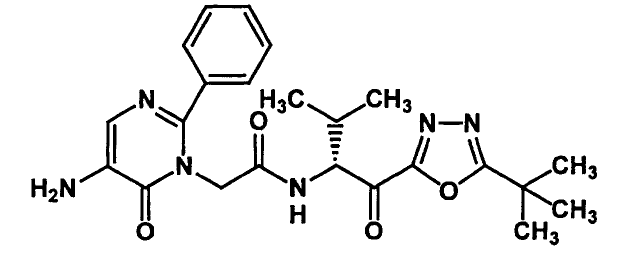
Compound (29): 2-[6-oxo-2-phenyl-1,6-dihydro-1-pyrimidinyl]-N-[(1S)-1-[[5-(t-butyl)-1,3,4-oxadiazole-2-yl]carbonyl]-2-methylpropyl]acetamide (CAS Registry No. 208848-14-0), 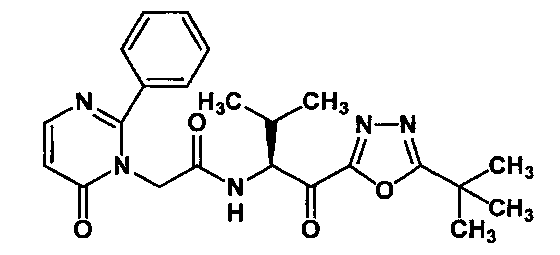
Compound (30): 2-[6-oxo-2-phenyl-1,6-dihydro-1-pyrimidinyl]-N-[(1R)-1-[[5-(t-butyl)-1,3,4-oxadiazole-2-yl]carbonyl]-2-methylpropyl]acetamide (CAS Registry No. 208848-09-3), 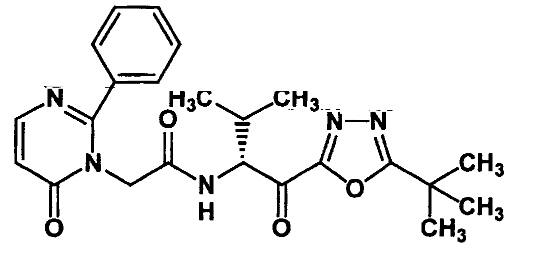
Compound (31): 2-[5-amino-6-oxo-2-(4-fluorophenyl)-1,6-dihydro-1-pyrimidinyl]-N-[1-[[5-(t-butyl)-1,3,4-oxadiazole-2-yl]carbonyl]-2-methylpropyl]acetamide (CAS Registry No. 208848-16-2), 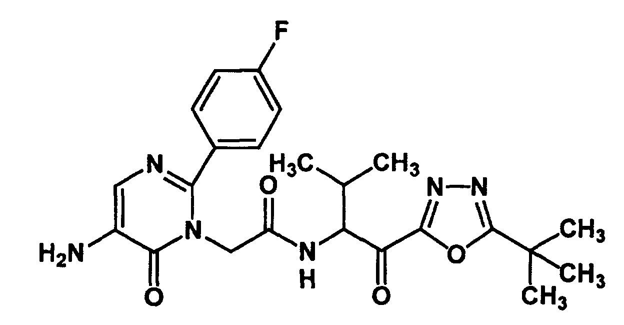
Compound (32): 2-[6-oxo-2-(4-fluorophenyl)-1,6-dihydro-1-pyrimidinyl]-N-[(1S)-1-[[5-(1-methylcyclopropyl)-1,3,4-oxadiazole-2-yl]carbonyl]-2-methylpropyl]acetamide (CAS Registry No. 208848-17-3), 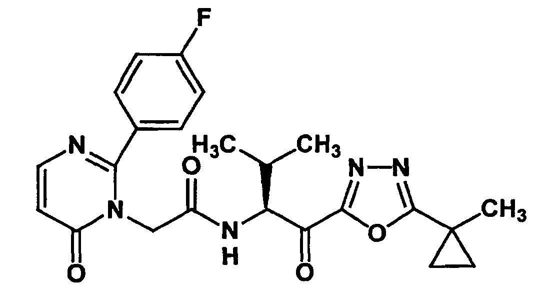
Compound (33): 2-[6-oxo-2-phenyl-1,6-dihydro-1-pyrimidinyl]-N-[(1S)-1-[[5-(1-methylcyclopropyl)-1,3,4-oxadiazole-2-yl]carbonyl]-2-methylpropyl]acetamide (CAS Registry No. 208848-18-4), 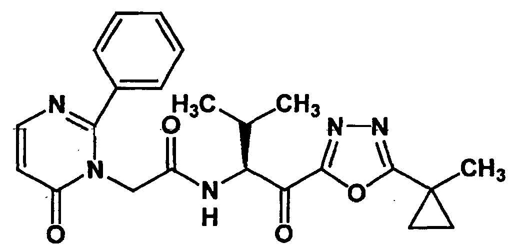
Compound (34): 2-[5-amino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (CAS Registry No. 208848-19-5), 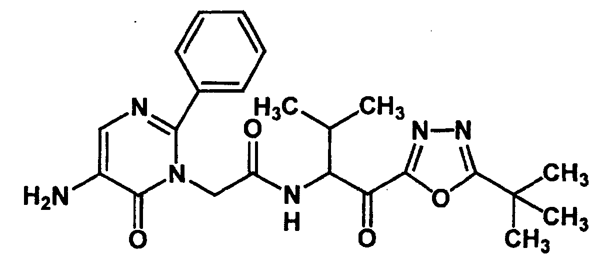
Compound (35): 2-[6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (CAS Registry No. 208848-20-8), 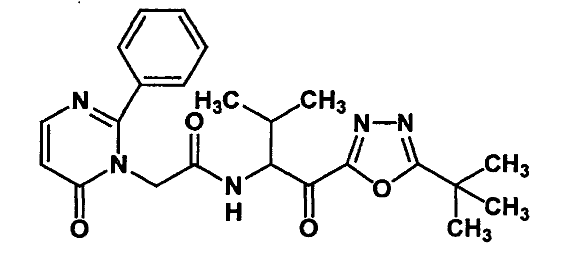
Compound (36): 2-[5-amino-6-oxo-2-(pyridine-3-yl)-1,6-dihydro-1-pyrimidinyl]-N-[(1S)-1-[[5-(t-butyl)-1,3,4-oxadiazole-2-yl]carbonyl]-2-methylpropyllacetamide (CAS Registry No. 208847-96-5), 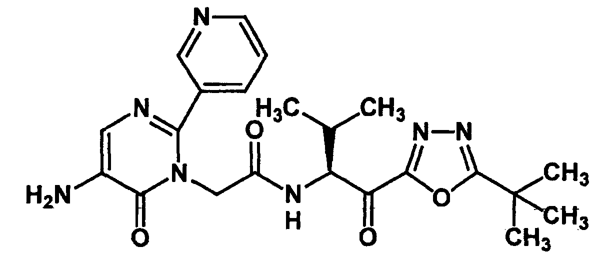
Compound (37): 2-[5-amino-6-oxo-2-(pyridine-3-yl)-1,6-dihydro-1-pyrimidinyl]-N-[(1S)-1-[[5-(α,α-dimethylbenzyl)-1,3,4-oxadiazole-2-yl]carbonyl]-2-methylpropyl]acetamide (CAS Registry No. 208847-99-8), 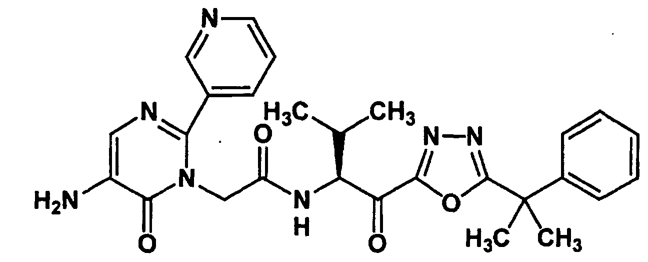
Compound (38): 2-[5-amino-6-oxo-2-(4-fluorophenyl)-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide (CAS Registry No. 251958-28-8), 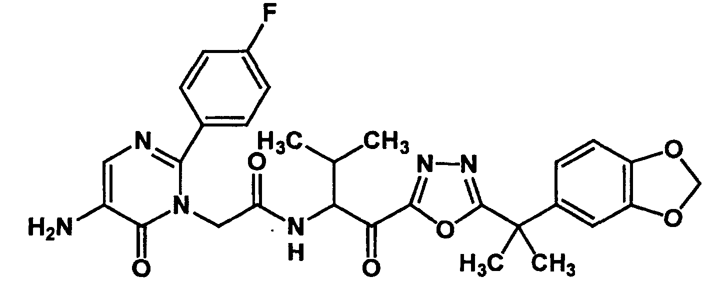
Compound (39): 2-[5-amino-6-oxo-2-phenyl-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide (CAS Registry No. 251958-31-3), 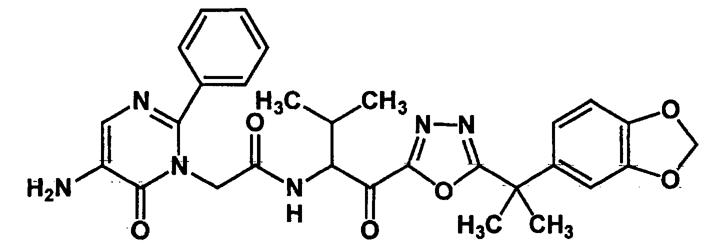
Compound (40): 2-[6-oxo-2-(4-fluorophenyl)-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide (CAS Registry No. 251958-33-5), 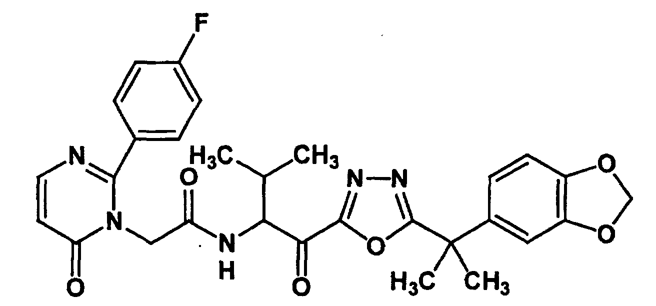
Compound (41): 2-[6-oxo-2-phenyl-1,6-dihydro-1-pyrimidinyl]-N-[1-[[5-(α,α-dimethylbenzyl)-1,3,4-oxadiazole-2-yl]carbonyl]-2-methylpropyl]acetamide (CAS Registry No. 251958-32-4), 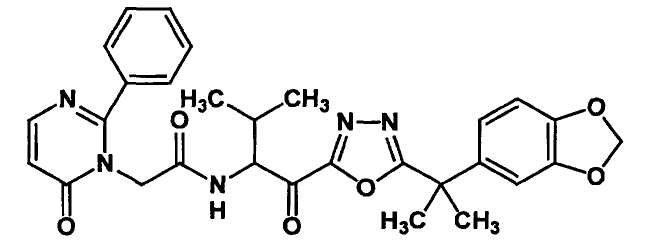
Compound (42): 2-[6-oxo-2-(4-fluorophenyl)-1,6-dihydro-1-pyrimidinyl]-N-[1-[[5-(α,α-dimethylbenzyl)-1,3,4-oxadiazole-2-yl]carbonyl]-2-methylpropyl]acetamide (CAS Registry No. 251958-34-6), 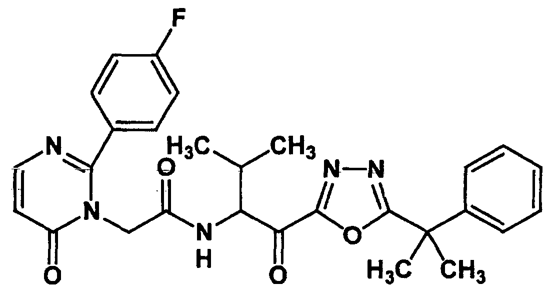
Compound (43): 2-[6-oxo-2-phenyl-1,6-dihydro-1-pyrimidinyl]-N-[1-[[5-(α,α-dimethylbenzyl)-1,3,4-oxadiazole-2-yl]carbonyl]-2-methylpropyl]acetamide (CAS Registry No. 251958-35-7), 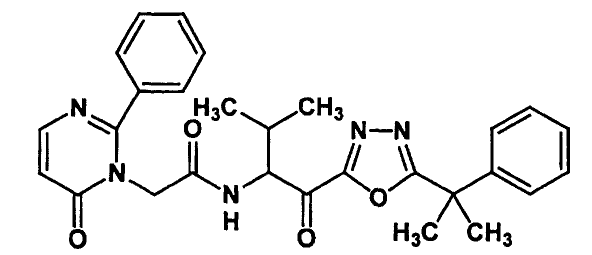
Compound (44): Methoxycarbonyl-L-valyl-N-[(1S)-1-[[5-(t-butyl)-1,3,4-oxadiazole-2-yl]carbonyl]-2-methylpropyl]-L-prolinamide (CAS Registry No. 251958-25-5) 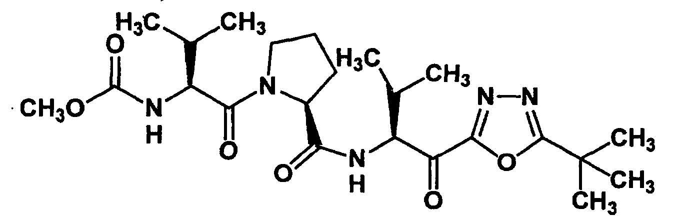
Compound (45): Methoxycarbonyl-L-valyl-N-[(1S)-1-[[5-(α,α-dimethylbenzyl)-1,3,4-oxadiazole-2-yl]carbonyl]-2-methylpropyl]-L-prolinamide (CAS Registry No. 251958-26-6), 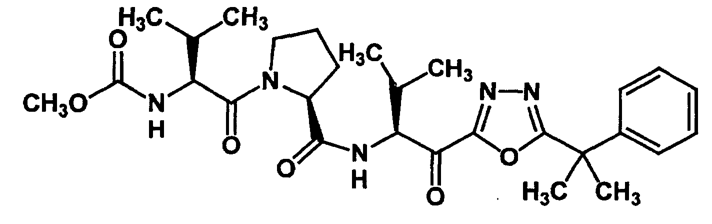
Compound (46): Methoxycarbonyl-L-valyl-N-[(1S)-1-[[5-(α,α-dimethyl-3,4-methylenedioxybenzyl)-1,3,4-oxadiazole-2-yl]carbonyl]-2-methylpropyl]-L-prolinamide (CAS Registry No. 251958-27-7). 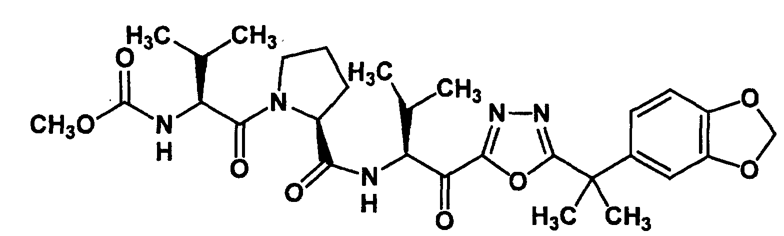
Compound (47): 2-[5-methoxycarbonylamino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (CAS Registry No. 251958-36-8), 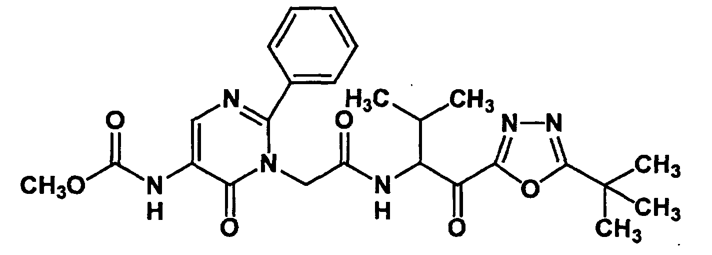
Compound (48): 1-[(3S)-(2-(t-butoxycarbonyl)-1,2,3,4-tetrahydroisoquinoline-3-yl]-N-[(1S)-1-[[5-(t-butyl)-1,3,4-oxadiazole-2-yl]carbonyl]-2-methylpropyl]carboxamide (CAS Registry No. 251540-43-9), 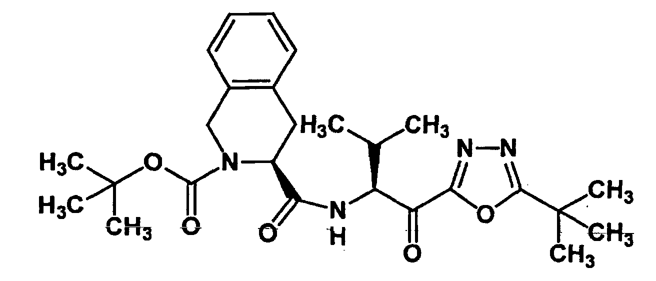
Compound (49): 1-[(3S)-(2-(1-methylethoxycarbonyl)-1,2,3,4-tetrahydroisoquinoline-3-yl]-N-[(1S)-1-[[5-(t-butyl)-1,3,4-oxadiazole-2-yl]carbonyl]-2-methylpropyl]carboxamide (CAS Registry No. 251540-44-0), 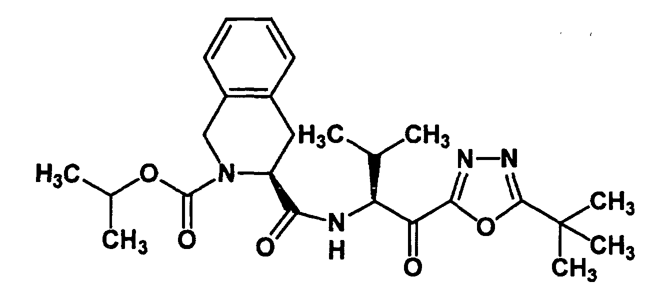
Compound (50): 1-[(3S)-(2-(imidazole-5-ylcarbonyl)-1,2,3,4-tetrahydroisoquinoline-3-yl]-N-[(1S)-1-[[5-(t-butyl)-1,3,4-oxadiazole-2-yl]carbonyl]-2-methylpropyl]carboxamide (CAS Registry No. 251540-45-1), 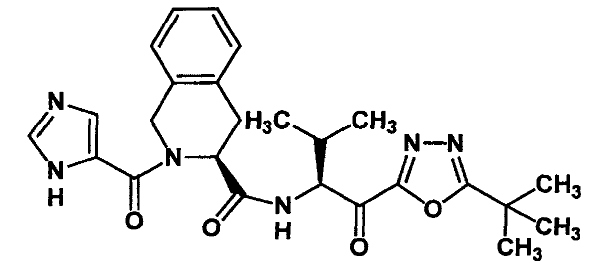
Compound (51): 1-[(3S)-(2-[(1S)-1-(methoxycarbonylamino)-2-methylpropylcarbonyl]-1,2,3,4-tetrahydroisoquinoline-3-yl]-N-[(1S)-1-[[5-(t-butyl)-1,3,4-oxadiazole-2-yl]carbonyl]-2-methylpropyl]carboxamide (CAS Registry No. 251540-46-2), 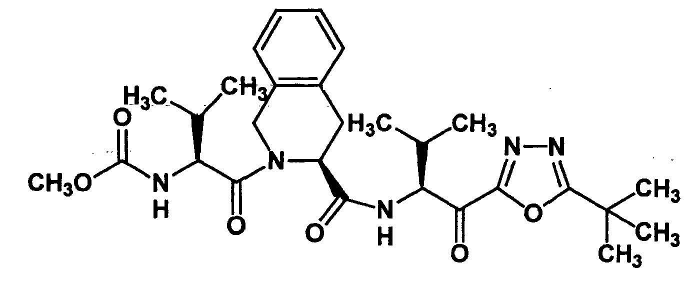
Compound (52): 1-[(3S)-(2-[(1S)-1-(pyridine-3-ylcarbonylamino)-2-methylpropylcarbonyl]-1,2,3,4-tetrahydroisoquinoline-3-yl]-N-[(1S)-1-[[5-(t-butyl)-1,3,4-oxadiazole-2-yl]carbonyl]-2-methylpropyl]carboxamide (CAS Registry No. 251540-47-3), 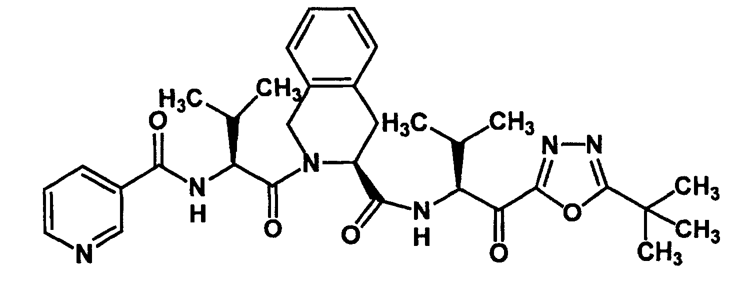
Compound (53): 1-[(2S)-1-(t-butoxycarbonyl)-2,3-dihydroindole-2-yl]-N-[(1S)-1-[[5-(t-butyl)-1,3,4-oxadiazole-2-yl]carbonyl]-2-methylpropyl]carboxamide (CAS Registry No. 251540-48-4), 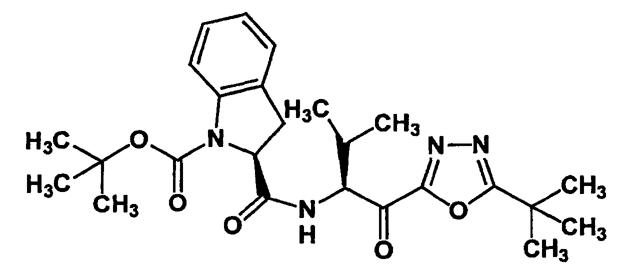
Compound (54): 1-[(2S)-1-(2-methylethoxycarbonyl)-2,3-dihydroindole-2-yl]-N-[(1S)-1-[[5-(t-butyl)-1,3,4-oxadiazole-2-yl]carbonyl]-2-methylpropyl]carboxamide (CAS Registry No. 251540-49-5), 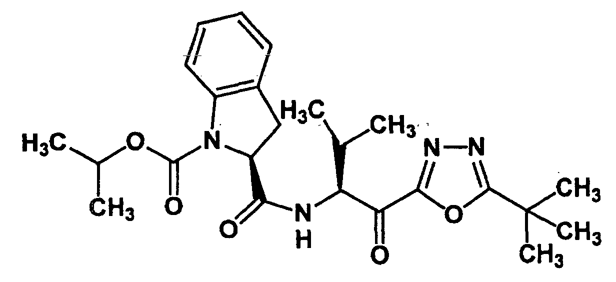
Compound (55): 1-[(2S)-1-(imidazole-5-ylcarbonyl)-2,3-dihydroindole-2-yl]-N-[(1S)-1-[[5-(t-butyl)-1,3,4-oxadiazole-2-yl]carbonyl]-2-methylpropyl]carboxamide (CAS Registry No. 251540-50-8), 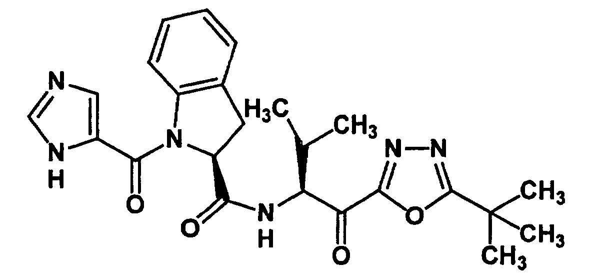
Compound (56): 1-[(2S)-1-[(1S)-1-(methoxycarbonylamino)-2-methylpropylcarbonyl]-2,3-dihydroindole-2-yl]-N-[(1S)-1-[[5-(t-butyl)-1,3,4-oxadiazole-2-yl]carbonyl]-2-methylpropyl]carboxamide (CAS Registry No. 251540-51-9), 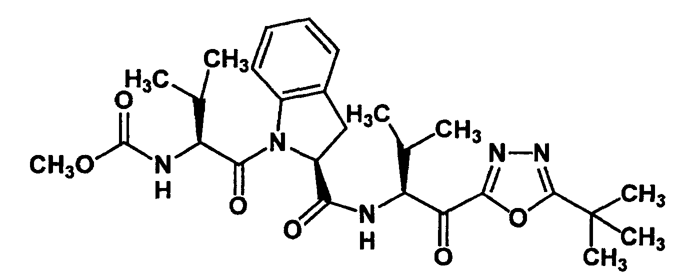
Compound (57): 1-[(2S)-1-[(1S)-1-(pyridine-3-ylcarbonylamino)-2-methylpropylcarbonyl]-2,3-dihydroindole-2-yl]-N-[(1S)-1-[[5-(t-butyl)-1,3,4-oxadiazole-2-yl]carbonyl]-2-methylpropyl]carboxamide (CAS Registry No. 251540-52-0) or 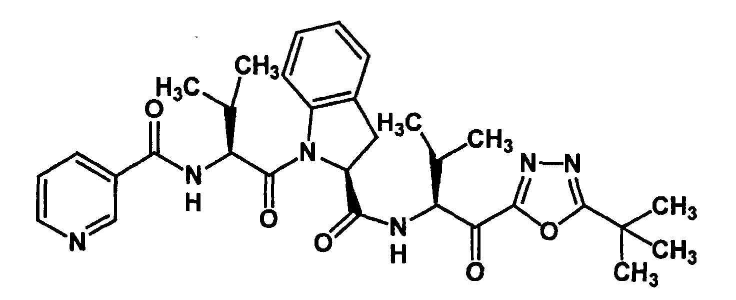
Compound (58): 2-[5-methoxycarbonylamino-6-oxo-2-phenyl-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide.

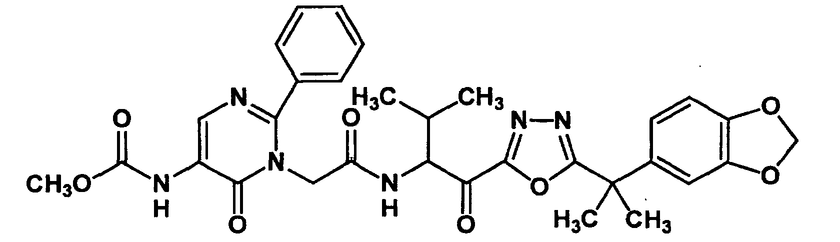

More desirable compounds are
2-[5-amino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (compound 34),
Methoxycarbonyl-L-valyl-N-[(1S)-1-[[5-(t-butyl)-1,3,4-oxadiazole-2-yl]carbonyl]-2-methylpropyl]-L-prolinamide (compound 44),
Methoxycarbonyl-L-valyl-N-[(1S)-1-[[5-(α,α-dimethyl-3,4-methylenedioxybenzyl)-1,3,4-oxadiazole-2-yl]carbonyl]-2-methylpropyl]-L-prolinamide (compound 46),
2-[5-methoxycarbonylamino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide, (compound 47) or
2-[5-methoxycarbonylamino-6-oxo-2-phenyl-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide (compound 58).

The most desirable compound is 2-[5-amino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (compound 34).

Compounds represented by the formula (I) used for the present invention may be used in the form of the nontoxicity salt allowed in pharmacology. As for these salts, the ones of the soluble without toxicity are desirable.

As a suitable salt, the inorganic acid salt such as hydrochloride, hydrogen bromide salt, sulfate, phosphate salts or nitrates, or the organic acid salt such as acetates, trifluoroacetates, lactic acid salts, tartrate salts, malates, oxalates, fumarates, maleates, citrates, benzoate salts, methanesulfonates, the ethane sulfonic acid salts, benzenesulfonates, toluenesulfonates, isethionic acid salts, glucuronate salts or gluconates are enumerated. Moreover, compounds represented by the formula (I) or the salts can be converted into the hydrate by a well-known method.

In the present invention, isomers include all isomers as long as it does not especially direct it. For instance, the one of the straight chain and the one of the branched-chain are included in alkyl, alkenyl, alkynyl group and the alkylene. In addition, all isomers (E, Z, cis and transformer body) in double bond, ring, condensed ring, isomer (R, S body, α, β body, enantiomer, and diastereomer) by existence of asymmetric carbons, etc., the optical isomer (D, L, d, and I body) with optical rotation, polarity body (high polarity body and low polarity body) separated by the chromatographic, balanced compounds, compounds of these arbitrary ratio and racemic mixtures are included in the present invention.

In addition, all the crystal forms where it can be taken are included in the compound shown by the formula (I) used by the present invention.

### BRIEF DESCRIPTION OF THE DRAWINGS

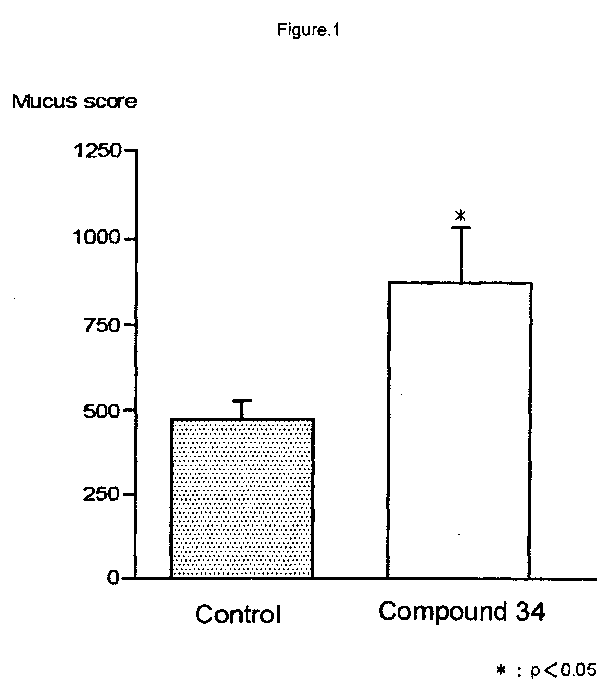

Figure 1 shows the result of evaluating the augmentative mucus secretion in guinea pig trachea as mucus score by PAS-Alcian Blue dye when compound 34 is administered

### THE BEST FORM TO EXECUTE INVENTION

The suppressive effect of compounds presented in the present invention on the airway mucus secretion was proven by the following experiments.

### Suppressive effect on augmentative secretion by antigen challenge

### [Experimental method]

Egg albumin (Ovalbumin:OA) (70 mg/kg) is administered and sensitized to guinea pig into the peritoneal cavity on the first experiment day and 8th day. The test drug (100 mg/kg) was oral administered to the sensitized guinea pig on 22nd experiment day. 5%OA was inhaled for 2 minutes 30 minutes after the test drug was administered, and the trachea was removed 10 minutes later. It was dyed with PAS-Alcian Blue, and the augmentative mucus secretion was evaluated according to the level of the decrease in mucus score.

As a test drug, 2-[5-amino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (Compound 34) was used.

Figure 1 shows the result.

### INDUSTRIAL APPLICABILITY

Because compound 34 intentionally suppressed mucus score in this model, it was understood that compounds of the present invention could have a strong suppressive effect on the augmentative mucus secretion. Therefore, it could be judged that compounds represented by the formula (I) are effective in diseases with the augmentative airway mucus secretion such as asthma.

### [Toxicity]

It could be judged that the toxicity of compounds represented by the formula (I) used for the present invention are very low, and they are very safe to use them as a medicine.

### [Application to medicine]

Because compounds represented by the formula (I) used for the present invention have the inhibitory action for the augmentative airway mucus secretion in animal including human, especially in human, they are useful for treatment and/or prevention of diseases with the augmentative airway mucus secretion such as asthma.

To use compounds of the present invention, the nontoxicity salts or the hydrates for the above purpose, they are usually administered as the form of the oral or non-oral systemically or locally.

A dosage is different depending on age, weight, symptom, therapeutic effect, administration mode, and processing time, etc., and they are orally administered once or several times a day within the range of a dosage from 1mg to 1000mg for adult, or are parenterally administered (Intravenous administration, desirably) within the range of a dosage from 0.1mg to 100mg or are continually intravenously administered for 1 hour to 24 hours a day.

Because a dosage changes according to various conditions as the above, it might be enough in less amount than the above dosage or might be necessary beyond the pale.

When compounds of the present invention are administered, they are used as solid medicines for taking for the oral administration, the liquid medicine for taking, injection drugs for parenteral administration, external preparations, inhalants, and suppositories, etc.

The tablet, the pill, the capsule, the powder, and the granule, etc. are included in the solid medicine for taking for the oral administration. A hard capsule and a soft capsule are included in the capsule.

One or more activators in such the solid medicine for taking is/are itself/themselves or are mixed with vehicles (lactose, mannitol, glucose, microcrystalline cellulose, and starch, etc.), binders (hydroxypropylcellulose, polyvinylpyrrolidone, and magnesium aluminometasilicate, etc.), disintegrators (cellulose glycolic acid calcium etc.), glidants (magnesium stearate etc.), stabilizers or solubilizers (glutamate and aspartate, etc.), etc. and are used as making to pharmaceutical preparations according to the usual method. Moreover, if necessary, they may be coated with coating materials (saccharose gelatin, hydroxypropylcellulose, and hydroxypropylmethylcellulose phthalate, etc.) or may be coated in the layer of two or more. In addition, capsules of materials absorbed like gelatin are included.

Liquid medicines for taking for the oral administration contain the solution, the suspension, the emulsion, the syrup drug, and the elixir, etc. allowed as medicines.

One or more activators in such these liquid medicines is/are dissolved, suspended or emulsified into diluents (purified water, ethanol or those mixing liquids, etc.) used generally. In addition, such this liquid medicine may contain penetrants, suspending agents, emulsifying agents, sweeteners, flavor medicines, aromatic substances, preservatives and buffers, etc.

Injections for parenteral administration include solid injection drugs used with being dissolved or being suspended into liquor, suspension, latex or time of use solvent. Injections are used with being dissolved, suspended or emulsified one or more activators into solvent. As solvents, distilled water for injection, physiological salt solution, vegetable oil, propylene glycol, polyethylene glycol or alcohol group, etc. such as ethanol or the mixture is used. In addition, this injections may contain stabilizers, solubilizers (glutamate, aspartate, and polysorbate 80 (registered trademark), etc.), suspending agents, emulsifying agents, medicines of making to aponia, buffers or preservatives, etc. These are manufactured and prepared by sterilized or aseptic manipulation in the final process. Moreover, it is possible to use them by manufacturing the sterilized solid medicine such as freeze-drying goods, and then dissolving to distilled water for injection or other solvents made to the sterility or the sterility before use.

Other preparations for parenteral administration, which contain one or more activators, include liquids for external use prescribed with usual methods, ointment drugs, coating drugs, inhalants, aerosols or pessaries for administering in suppository and vagina, etc.

Besides diluents generally used, aerosols may contain stabilizers like the sodium hydrogensulfite, buffers which gives isotonicity or isotonic medicines such as sodium chloride and, sodium citrate or citrates. The manufacturing method of aerosol has been described in detail, for example, in the U.S. patent No.2,868,691 and No.3,095,355.

### Example 1 of preparation

100 tablets that contained 50mg of the active constituent in a tablet, which are compressed after mix with the following each elements by usual methods, were obtained.
· 2-[5-amino-6-oxo-2-(4-fluorophenyl)-1,6-dihydro-1-pyrimidinyl]-N-[1-(2-[5-tert-butyl-1,3,4-oxadiazole]carbonyl)-2-(R,S)-methylpropyl]acetamide 5.0g
· Carboxymethylcellulose calcium (disintegrator) 0.2g
· Magnesium stearate (lubricant) 0.1g
· Microcrystalline cellulose 4.7g

### Example 2 of preparation

100 ampoules that contained 20mg of the active constituent in a ampoule, which are sterilized by usual methods and be filled each 5ml to the ampoule followed by being freeze-dried by usual methods, were obtained.
· 2-[5-amino-6-oxo-2-(4-fluorophenyl)-1,6-dihydro-1-pyrimidinyl]-N-[1-(2-[5-tert-butyl-1,3,4-oxadiazole]carbonyl)-2-(R,S)-methylpropyl]acetamide 2.0g
· Mannitol 20g
· Distilled water 500ml

## Claims

1. Prophylactic and/or therapeutic drugs for diseases with the augmentative airway mucus secretion, which include five-member heterocyclic compounds represented by the formula (I) or these nontoxic salts as active principle, 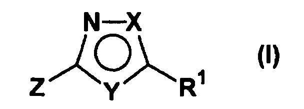 (wherein Z represents the group to contain α-aminocarbonyl group that carbonyl carbon atom combines with carbon atom of heterocyclic group through covalent bond,
R¹ represents (1) alkyl, alkenyl or alkynyl groups, with the proviso that these groups may be substituted with one or more groups chosen from (a)halogen atom, (b)hydroxy, (c)cyano, (d)nitro, (e)haloalkyl, (f)alkylamino, (g)dialkylamino (h)alkoxy, (I)haloalkoxy, (j)carboxy, (k)carboalkoxy, (I)alkylcaroxamide, (m)arylcaroxamide or (n)-O-(C5-C6) aryl groups,
(2)hydroxy,
(3)amino,
(4)alkylamino,
(5)dialkylamino, or
(6)cycloalkyl, alkylcycloalkyl, alkenylcycloalkyl, cycloalkenyl, alkylcycloalkenyl, alkenylcycloalkenyl, (C5-C12)aryl, (C5-C12)arylalkyl, (C5-C12)arylalkenyl, condensable (C5-C12)aryl-cycloalkyl or alkyl condensable (C5-C12)aryl-cycloalkyl, with the proviso that these groups may be substituted with groups chosen from (a)halogen atom, (b)hydroxy, (c)cyano, (d)nitro, (e)haloalkyl, (f)amino, (g)aminoalkyl, (h)dialkylamino, (i)alkyl, (j)alkenyl, (k)alkylenedioxy, (l)alkynyl, (m)alkoxy, (n)haloalkoxy, (o)carboxy, (p)carboalkoxy, (q)carboxamide, (r)(C5-C6)aryl, (s)-O-(C5-C6)aryl, (t)arylcarboxamide, (u)alkylthio or (v)haloaklylthio that may include 1-4 of. hetero atoms chosen from nitrogen, oxygen or sulfur atom, respectively,
X and Y each independently represent oxygen, sulfur, nitrogen or atom,
which nitrogen atom may be substituted with groups chosen from
(1)alkyl or alkenyl that may be substituted with 1-3 of halogen atom,
(2)alkynyl,
(3)(C5-C6)aryl, arylalkyl, arylalkenyl that may include 1-3 of hetero atoms chosen from nitrogen, oxygen or sulfur atom or may be substituted with groups chosen from (a)halogen atom, (b)cyano, (c)nitro, (b)hydroxy, (e)haloalkyl, (f)amino, (g)aminoalkyl, (h)dialkylamino, (i)alkyl, (j)alkenyl, (k)alkynyl, (l)alkoxy, (m)haloalkoxy, (n)carboxy, (o)carboalkoxy, (p)carboxamide, (q)arylcarboxamide, (r)alkylthio or (s)haloaklylthio).

2. Prophylactic and/or therapeutic drugs described in claim 1 wherein Z is represented by the formula (I-1), 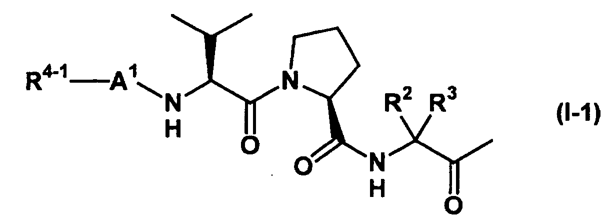 (wherein R² and R³ each independently represent alkyl, alkenyl, -RCOR', -RCOOR', -RNR'R"R⁰, or -RC(O)NR'R"[wherein R represents alkyl or alkenyl, R', R" and R⁰ each independently represent hydrogen atom, alkyl, alkenyl, cycloalkyl or (C5-C6)aryl.] with the proviso that the above groups optionally be substituted with hydrogen, 1-3 of halogen atom, hydroxy, sulfur atom, alkylthio, amino, alkylamino, dialkylamino, alkylguanidinyl, dialkylguanidinyl, guanidinyl or amidylguanidine; or cycloalkyl, alkylcycloalkyl, alkenylcycloalkyl, alkyl-oxyaryl, alkyl-thioaryl, alkyl-aminoaryl, (C5-C12)aryl, (C5-C12)arylalkyl or (C5-C12)arylalkenyl with the proviso that the above groups optionally include 1-4 of hetero atoms chosen from nitrogen, oxygen or sulfur atom and are substituted with halogen atoms, cyano, keto, nitro, hydroxy, haloalkyl, amino, aminoalkyl, dialkylamino, amidine, alkylamidine, dialkylamidine, alkyl, alkenyl, alkylenedioxy, alkynyl, alkoxy, haloalkoxy, carboxy, carboalkoxy, alkylcarboxamide, (C5-C6)aryl, -O-(C5-C6)aryl, arylcarboxamide, alkylthio or haloalkylthio,
A¹ represents direct coupling, -C(O)-, -NH-C(O)-, -S(O)₂-, -NH-S(O)₂-, -C(O)-, -C- or, for example, amino acids chosen from the following, amino acids aren't limited to these;
proline, isoleucine or cyclohexylalanine; cysteine, phenylalanine, homophenylalanine, dehydrophenylalanine, indoline-2-carboxylic acid with the proviso that the above amino acids optionally be substituted with alkyl, alkenyl or phenyl, and that sulfur atom optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthio; tetrahydroisoquinoline-2-carboxylic acid which optionally be substituted in alkyl, alkenyl or phenyl, with the proviso that these groups optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthiotryptophan; tryptophane, tyrosine, serine, threonine; histidine, methionine, valine, norvaline, norleucine or octahydroindole-2-carboxylic acid with the proviso that these amino acids optionally be substituted with alkyl or aryl; asparagine, glutamine, ornithine or lysine with the proviso that nitrogen atoms in side chains of these amino acids optionally be substituted with alkyl, alkenyl, alkynyl, alkoxyalkyl, alkylthioalkyl, alkylaminoalkyl, dialkylaminoalkyl, carboxyalkyl, alkoxyalkyl, alkylthioalkyl, alkylaminoalkyl, dialkylaminoalkyl, carboxyalkyl, alkoxycarbonylalkyl, aryl, arylalkyl, cycloalkyl, alkylcycloalkyl, condensed aryl-cycloalkyl or alkyl condensed aryl-cycloalkyl and optionally include one or more hetero atoms chosen from nitrogen, oxygen or sulfur atom,
R⁴⁻¹ represents cycloalkyl, alkylcycloalkyl, (C5-C12)aryl, (C5-C12)arylalkyl, condensed (C5-12)aryl-cycloalkyl or condensed alkyl(C5-C12)aryl-cycloalkyl with the proviso that these groups optionally include hydrogen atom, alkyl, alkenyl or alkynyl, one or more hetero atoms chosen from nitrogen, oxygen or sulfur atom, and optionally be substituted with alkyl, alkenyl, alkynyl, halogen atom, cyano, nitro, hydroxy, haloalkyl, alkoxy, amino, aminoalkyl, dialkylamino, carboxy, haloalkoxy, carboalkoxy, alkylcarboxamide, aryl, arylalkyl, arylcarboxamide, alkylthio or haloalkylthio).

3. Prophylactic and/or therapeutic drugs described in claim 1 wherein Z is represented by the formula (I-2) 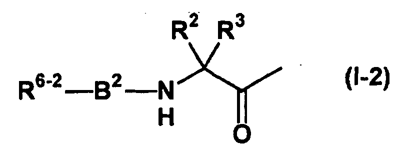 (wherein R² and R³ represent the same meanings as aforesaid;
B² represents -S(O)₂-, -C(O)-, -OC(O)- or -CH₂C(O)-;
R⁶⁻² represents the following groups; 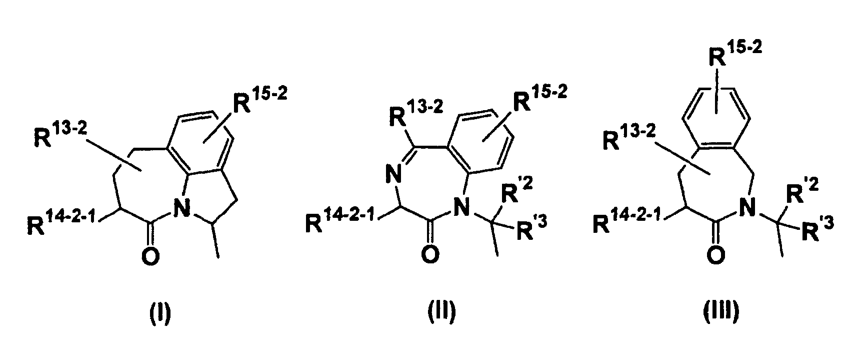 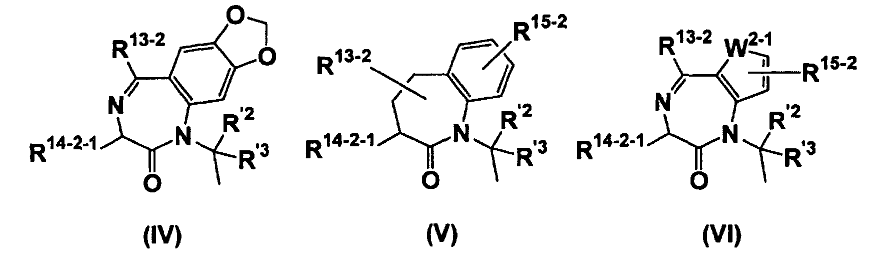 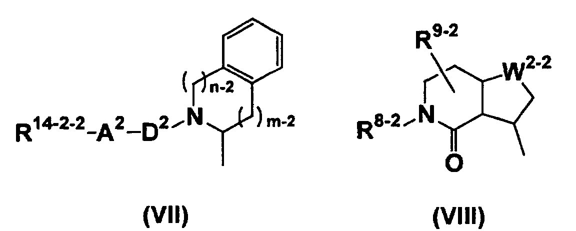 (wherein R'² and R'³ represent the same meanings as R² and R³;
R¹³⁻² represents aryl, arylalkyl, cycloalkyl, alkylcycloalkyl, condensed aryl-cycloalkyl, alkyl condensed aryl-cycloalkyl with the proviso that these groups optionally include hydrogen atom, alkyl, halogen atom, alkoxy, carboalkoxy, carboxy, alkylthio, amino, alkylamino, dialkylamino or one or more hetero atoms chosen from nitrogen, oxygen or sulfur atom, and optionally be substituted with halogen atom or alkyl;
R¹⁴⁻²⁻¹ represents aryl, arylalkyl, cycloalkyl, alkylcycloalkyl, condensed aryl-cycloalkyl, alkyl condensed aryl-cycloalkyl or aryloxycarboxamide with the proviso that these groups optionally include hydrogen atom, alkyl, alkenyl, amino, alkylamino, dialkylamino or one or more hetero atoms chosen from nitrogen, oxygen or sulfur atom, and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, arylalkylcarboxamide, alkylthio or haloalkylthio;
R¹⁵⁻² represents aryl, arylalkyl, cycloalkyl, alkylcycloalkyl, condensed aryl-cycloalkyl or alkyl condensed aryl-cycloalkyl with the proviso that these groups optionally include halogen atom, alkyl, halogen atom, alkoxy, carboalkoxy, carboxy, alkylthio, amino, alkylamino, dialkylamino or one or more hetero atoms chosen from nitrogen, oxygen or sulfur atom;
W²⁻¹ represents oxygen or sulfur atom; carbon or nitrogen atom that optionally be substituted with hydrogen atom, alkyl or aryl;
[m-2] represents 0 or 1;
[n-2] represents 0 or 1;
D² represents direct coupling or, for example, amino acids chosen from the following, amino acids aren't limited to these;
proline, isoleucine or cyclohexylalanine; cysteine, phenylalanine, homophenylalanine, dehydrophenylalanine, indoline-2-carboxylic acid with the proviso that the above amino acids optionally be substituted with alkyl, alkenyl or phenyl, and that sulfur atom optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthio; tetrahydroisoquinoline-2-carboxylic acid which optionally be substituted in alkyl, alkenyl or phenyl, with the proviso that these groups optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthiotryptophan; tryptophane, tyrosine, serine, threonine; histidine, methionine, valine, norvaline, norleucine or octahydroindole-2-carboxylic acid with the proviso that these amino acids optionally be substituted with alkyl or aryl; asparagine, glutamine, ornithine or lysine with the proviso that nitrogen atoms in side chains of these amino acids optionally be substituted with alkyl, alkenyl, alkynyl, alkoxyalkyl, alkylthioalkyl, alkylaminoalkyl, dialkylaminoalkyl, carboxyalkyl, alkoxyalkyl, alkylthioalkyl, alkylaminoalkyl, dialkylaminoalkyl, carboxyalkyl, alkoxycarbonylalkyl, aryl, arylalkyl, cycloalkyl, alkylcycloalkyl, condensed aryl-cycloalkyl or alkyl condensed aryl-cycloalkyl and optionally include one or more hetero atoms chosen from nitrogen, oxygen or sulfur atom;
A² represents direct coupling, -C(O)-, -NH-C(O)-, -S(O)₂-, -NH-S(O)₂-, -C(O)- or -C-;
R¹⁴⁻²⁻² represents aryl, arylalkyl, cycloalkyl, alkylcycloalkyl, condensed aryl-cycloalkyl or alkyl condensed aryl-cycloalkyl with the proviso that these groups optionally include hydrogen atom, alkyl, alkenyl, amino, alkylamino, dialkylamino or one or more hetero atoms chosen from nitrogen, oxygen or sulfur atom, and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, alkylthio or haloalkylthio;
W²⁻² represents sulfur or oxygen atom;
R⁸⁻² represents alkylamino dialkylamino or amino;
R⁹⁻² represents hydrogen atom, alkyl or halogen atom)).

4. Prophylactic and/or therapeutic drugs described in claim 1 wherein Z is represented by the formula (I-3), 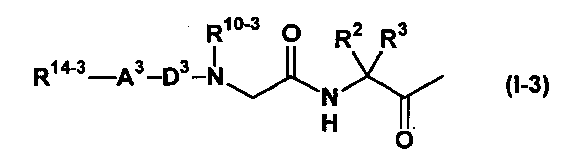 (wherein R² and R³ represent the same meanings as aforesaid,
R¹⁰⁻³ represents (C5-C6)aryl, (C5-C6)arylalkyl, (C5-C6)arylalkenyl, cycloalkyl, condensed aryl-cycloalkyl with the proviso that these groups optionally include one or more hetero atoms chosen from nitrogen, sulfur or non-peroxideone oxygen atom, and optionally be substituted with halogen atom, cyano, nitro, haloalkyl, amino, aminoalkyl, dialkylamino, alkyl, alkenyl, alkoxy, haloalkoxy, carboxy, carboalkoxy, alkylcarboxamide, alkylthio or haloalkylthio;
D³ represents direct coupling, -C(O)- or, for example, amino acids chosen from the following, amino acids aren't limited to these;
proline, isoleucine or cyclohexylalanine; cysteine, phenylalanine, homophenylalanine, dehydrophenylalanine, indoline-2-carboxylic acid with the proviso that the above amino acids optionally be substituted with alkyl, alkenyl or phenyl, and that sulfur atom optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthio; tetrahydroisoquinoline-2-carboxylic acid which optionally be substituted in alkyl, alkenyl or phenyl, with the proviso that these groups optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthiotryptophan; tryptophane, tyrosine, serine, threonine; histidine, methionine, valine, norvaline, norleucine or octahydroindole-2-carboxylic acid with the proviso that these amino acids optionally be substituted with alkyl or aryl; asparagine, glutamine, ornithine or lysine with the proviso that nitrogen atoms in side chains of these amino acids optionally be substituted with alkyl, alkenyl, alkynyl, alkoxyalkyl, alkylthioalkyl, alkylaminoalkyl, dialkylaminoalkyl, carboxyalkyl, alkoxyalkyl, alkylthioalkyl, alkylaminoalkyl, dialkylaminoalkyl, carboxyalkyl, alkoxycarbonylalkyl, aryl, arylalkyl, cycloalkyl, alkylcycloalkyl, condensed aryl-cycloalkyl or alkyl condensed aryl-cycloalkyl and optionally include one or more hetero atoms chosen from nitrogen, oxygen or sulfur atom;
A³ represents direct coupling, -C(O)-, -NH-C(O)-, -S(O)₂-, -NH-S(O)₂-, -C(O)-, -S(O)2-NH-, OC(0)NH-, -OC(O)- or -C-;
R¹⁴⁻² and R¹⁴⁻²⁻¹ represent the same meanings as aforesaid).

5. Prophylactic and/or therapeutic drugs described in claim 1 wherein Z is represented by the formula (I-4), 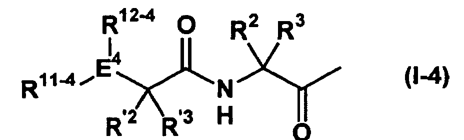 (wherein R², R³ R'² and R'³ represent the same meanings as aforesaid,
R¹¹⁻⁴, R¹²⁻⁴ and E⁴ are constituted of monocyclic or dicyclic ring with the proviso that these rings include 5-10 atoms chosen from carbon, nitrogen, sulfur or oxygen atom and include one or more keto groups and optionally be substituted with halogen atom, cyano, nitro, haloalkyl, amino, aminoalkyl, dialkylamino, alkyl, alkenyl, alkynyl, alkoxy, haloalkoxy, carboxy, carboalkoxy, alkylcarboxamide, alkylthio or haloalkylthio; and represent cycloalkyl, alkylcycloalkyl, alkenylcycloalkyl, (C5-C12)aryl, (C5-C12)arylalkyl, ((C5-C12)arylalkyl)OC(O)NH- or (C5-C12)arylalkyl with the proviso that these groups optionally include one or more hetero atoms chosen from nitrogen, sulfur or non-peroxideone oxygen atom, and optionally be substituted with halogen atom, cyano, nitro, haloalkyl, amino, aminoalkyl, dialkylamino, alkyl, alkenyl, alkoxy, haloalkoxy, carboxy, carboalkoxy, -C(O)O(alkyl), -C(O)(alkyl), alkylcarboxamide, alkylthio or haloalkylthio).

6. Prophylactic and/or therapeutic drugs described in claim 5 wherein R¹¹⁻⁴, R¹²⁻⁴ and E⁴ are represented by the formula (I-4), 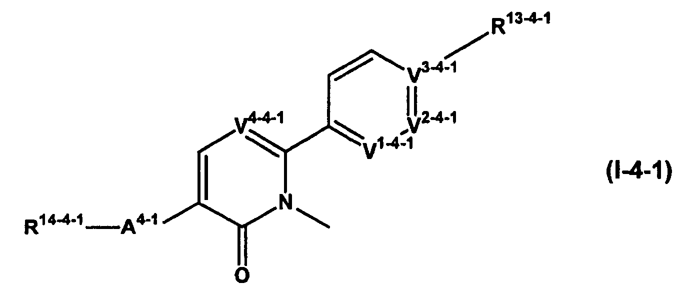 (wherein A⁴⁻¹ represents the same meanings as A³, V¹⁻⁴⁻¹, V²⁻⁴⁻¹, V³⁻⁴⁻¹ and V⁴⁻⁴⁻¹ each independently represent carbon or nitrogen atom, in case V³⁻⁴⁻¹ represents carbon atom, R¹³⁻⁴⁻¹ represents hydrogen atom, alkyl, halogen atom, alkoxy, carboalkoxy, carboxy, alkylthio, amino, alkylamino, dialkylamino; aryl, arylalkyl, cycloalkyl, alkylcycloalkyl, condensed aryl-cycloalkyl or alkyl condensed aryl-cycloalkyl with the proviso that these groups optionally include one or more hetero atoms chosen from nitrogen, sulfur or oxygen atom, and optionally be substituted with halogen atom or alkyl;
R¹⁴⁻⁴⁻¹ represents hydrogen atom, alkyl, alkenyl, amino, alkylamino or dialkylamino; aryl, arylalkyl, cycloalkyl, alkylcycloalkyl, condensed aryl-cycloalkyl or alkyl condensed aryl-cycloalkyl, arylalkylcarbonyl or arylalkylcarboxamide with the proviso that these groups optionally include one or more hetero atoms chosen from nitrogen, sulfur or oxygen atom, and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, alkylthio or haloalkylthio).

7. Prophylactic and/or therapeutic drugs described in claim 1 wherein compounds are selected from;
(1) 2-[5-amino-6-oxo-2-(4-fluorophenyl)-1,6-dihydro-1-pyrimidinyl]-N-[(1S)-1-[[5-(3-methylbenzyl)-1,3,4-oxadiazole-2-yl]carbonyl]-2-methylpropyl]acetamide,
(2) (2S,5S)-4-oxo-amino-5-1,2,4,5,6,7-hexahydro-N-[(1S)-1-[[5- 3-methylbenzyl)-1,3,4-oxadiazole-2-yl]carbonyl]-2-methylpropyl]azepino-[3,2,1]-indole-2-carboxamide,
(3) 2-[3[amino-2-oxo-5-phenyl-1,4-benzodiazepinyl]-N-1-[[5-(3-methylbenzyl)-1,3,4-oxadiazole-2-yl]carbonyl]-2-methylpropyl]acetamide,
(4) Methylsulfonyl-L-valyl-N-[(1S)-1-[[5-(3-methylbenzyl)-1,3,4-oxadiazole-2-yl]carbonyl]-2-methylpropyl]-L-prolinamide,
(5) 2-[4-(R)-isopropyl-2,5-imidazolidinedione-1-yl]-N-[(1S)-1-[[5-(3-methylbenzyl)-1,3,4-oxadiazole-2-yl]carbonyl]-2-methylpropyl]acetamide,
(6) Benzyloxycarbonyl-L-valyl-N-[(1S)-1-[[5-(3-methylbenzyl)-1,3,4-oxadiazole-2-yl]carbonyl]-2-methylpropyl]-L-prolinamide,
(7) 2-[3-(2-(morpholine-2-yl)ethyl)-4-phenyl-2,5- imidazolidinedione-1-yl]-N-[1-[[5-(3-methylbenzyl)-1,3,4-oxadiazole-2-yl]carbonyl]-2-methylpropyl] acetamide,
(8) 2-[4-methyl-4-(pyridine-2-yl)-2,5-imidazolidinedione-1-yl]-N-1-[[5-(3-methylbenzyl)-1,3,4-oxadiazole-2-yl]carbonyl]-2-methylpropyl] acetamide,
(9) 2-[(4S)-4-(2-methylpropyl)-2,5-imidazolidinedione-1-yl]-N-1-[[5-(3-methylbenzyl)-1,3,4-oxadiazole-2-yl]carbonyl]-2-methylpropyl]acetamide,
(10) 2-[5-amino-6-oxo-2-(4-fluorophenyl)-1,6-dihydro-1-pyrimidinyl]-N-[(1S)-1-[[5-(α,α-methylbenzyl)-1,3,4-oxadiazole-2-yl]carbonyl]-2-methylpropyl]acetamide,
(11) 2-[5-amino-6-oxo-2-(4-fluorophenyl)-1,6-dihydro-1-pyrimidinyl]-N-[(1S)-1-[[5-(α,α-dimethyl-3-methylbenzyl)-1,3,4-oxadiazole-2-yl]carbonyl]-2-methylpropyl]acetamide,
(12) 2-[5-amino-6-oxo-2-(4-fluorophenyl)-1,6-dihydro-1-pyrimidinyl]-N-[(1R)-1-[[5-(3-methylbenzyl)-1,3,4-oxadiazole-2-yl]carbonyl]-2-methylpropyl]acetamide,
(13) 2-[5-amino-6-oxo-2-(4-fluorophenyl)-1,6-dihydro-1-pyrimidinyl]-N-[(1S)-1-[[5-phenyl-1,3,4-oxadiazole-2-yl]carbonyl]-2-methylpropyl]acetamide,
(14) 2-[5-amino-6-oxo-2-(4-fluorophenyl)-1,6-dihydro-1-pyrimidinyl]-N-[(1S)-1-[[5-(t-butyl)-1,3,4-oxadiazole-2-yl]carbonyl]-2-methylpropyl]acetamide,
(15) 2-[5-amino-6-oxo-2-(4-fluorophenyl)-1,6-dihydro-1-pyrimidinyl]-N-[(1S)-1-[[5-(α,α-dimethyl-3,4-methylenedioxybenzyl)-1,3,4-oxadiazole-2-yl]carbonyl]-2-methylpropyl]acetamide,
(16). 2-[5-amino-6-oxo-2-(4-fluorophenyl)-1,6-dihydro-1-pyrimidinyl]-N-[(1S)-1-[[5-benzyl-1,3,4-oxadiazole-2-yl]carbonyl]-2-methylpropyl]acetamide,
(17) 2-[5-amino-6-oxo-2-(4-fluorophenyl)-1,6-dihydro-1-pyrimidinyl]-N-[(1S)-1-[[5-methyl-1,3,4-oxadiazole-2-yl]carbonyl]-2-methylpropyl]acetamide,
(18) 2-[5-amino-6-oxo-2-(4-fluorophenyl)-1,6-dihydro-1-pyrimidinyl]-N-[(1S)-1-[[5-(1-methylethyl)-1,3,4-oxadiazole-2-yl]carbonyl]-2-methylpropyl]acetamide,
(19) 2-[5-amino-6-oxo-2-(4-fluorophenyl)-1,6-dihydro-1-pyrimidinyl]-N-[(1S)-1-[[5-butyl-1,3,4-oxadiazole-2-yl]carbonyl]-2-methylpropyl]acetamide,
(20) 2-[5-amino-6-oxo-2-phenyl-1,6-dihydro-1-pyrimidinyl]-N-[(1S)-1-[[5-(t-butyl)-1,3,4-oxadiazole-2-yl]carbonyl]-2-methylpropyl]acetamide,
(21) 2-[5-amino-6-oxo-2-phenyl-1,6-dihydro-1-pyrimidinyl]-N-[(1S)-1-[[5-(α,α-dimethylbenzyl)-1,3,4-oxadiazole-2-yl]carbonyl]-2-methylpropyl]acetamide,
(22) 2-[5-amino-6-oxo-2-(4-fluorophenyl)-1,6-dihydro-1-pyrimidinyl]-N-[(1S)-1-[[5-(1-methylcyclopropyl)-1,3,4-oxadiazole-2-yl]carbonyl]-2-methylpropyl]acetamide,
(23) 2-[6-oxo-2-(4-fluorophenyl)-1,6-dihydro-1-pyrimidinyl]-N-[(1S)-1-[[5-(t-butyl)-1,3,4-oxadiazole-2-yl]carbonyl]-2-methylpropyl]acetamide,
(24) 2-[5-amino-6-oxo-2-(4-fluorophenyl)-1,6-dihydro-1-pyrimidinyl]-N-[(1R)-1-[[5-(t-butyl)-1,3,4-oxadiazole-2-yl]carbonyl]-2-methylpropyl]acetamide,
(25) 2-[5-amino-6-oxo-2-(4-fluorophenyl)-1,6-dihydro-1-pyrimidinyl]-N-[(1R)-1-[[5-(a,a-dimethylbenzyl)-1,3,4-oxadiazole-2-yl]carbonyl]-2-methylpropyl]acetamide,
(26) 2-[6-oxo-2-(4-fluorophenyl)-1,6-dihydro-1-pyrimidinyl]-N-[(1S)-1-[[5-(α,α-dimethylbenzyl)-1,3,4-oxadiazole-2-yl]carbonyl]-2-methylpropyl]acetamide,
(27) 2-[5-amino-6-oxo-2-phenyl-1,6-dihydro-1-pyrimidinyl]-N-[(1S)-1-[[5-(1-methylcyclopropyl)-1,3,4-oxadiazole-2-yl]carbonyl]-2-methylpropyl]acetamide,
(28) 2-[5-amino-6-oxo-2-phenyl-1,6-dihydro-1-pyrimidinyl]-N-[(1R)-1-[[5-(t-butyl)-1,3,4-oxadiazole-2-yl]carbonyl]-2-methylpropyl]acetamide,
(29) 2-[6-oxo-2-phenyl-1,6-dihydro-1-pyrimidinyl]-N-[(1S)-1-[[5-(t-butyl)-1,3,4-oxadiazole-2-yl]carbonyl]-2-methylpropyl]acetamide,
(30) 2-[6-oxo-2-phenyl-1,6-dihydro-1-pyrimidinyl]-N-[(1R)-1-[[5-(t-butyl)-1,3,4-oxadiazole-2-yl]carbonyl]-2-methylpropyl]acetamide,
(31) 2-[5-amino-6-oxo-2-(4-fluorophenyl)-1,6-dihydro-1-pyrimidinyl]-N-[1-[[5-(t-butyl)-1,3,4-oxadiazole-2-yl]carbonyl]-2-methylpropyl]acetamide,
(32) 2-[6-oxo-2-(4-fluorophenyl)-1,6-dihydro-1-pyrimidinyl]-N-[(1S)-1-[[5-(1-methylcyclopropyl)-1,3,4-oxadiazole-2-yl]carbonyl]-2-methylpropyl]acetamide,
(33) 2-[6-oxo-2-phenyl-1,6-dihydro-1-pyrimidinyl]-N-[(1S)-1-[[5-(1-methylcyclopropyl)-1,3,4-oxadiazole-2-yl]carbonyl]-2-methylpropyl]acetamide,
(34) 2-[5-amino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide,
(35) 2-[6-oxo-2-phenyl-1,6-dihydro-1-pyrimidinyl]-N-[1-[[5-(t-butyl)-1,3,4-oxadiazole-2-yl]carbonyl]-2-methylpropyl]acetamide,
(36) 2-[5-amino-6-oxo-2-(pyridine-3-yl)-1,6-dihydro-1-pyrimidinyl]-N-[(1S)-1-[[5-(t-butyl)-1,3,4-oxadiazole-2-yl]carbonyl]-2-methylpropyl]acetamide,
(37) 2-[5-amino-6-oxo-2-(pyridine-3-yl)-1,6-dihydro-1-pyrimidinyl]-N-[(1S)-1-[[5-(α,α-dimethylbenzyl)-1,3,4-oxadiazole-2-yl]carbonyl]-2-methylpropyl]acetamide,
(38) 2-[5-amino-6-oxo-2-(4-fluorophenyl)-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide,
(39) 2-[5-amino-6-oxo-2-phenyl-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide,
(40) 2-[6-oxo-2-(4-fluorophenyl)-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide,
(41) 2-[6-oxo-2-phenyl-1,6-dihydro-1-pyrimidinyl]-N-[1-[[5-(α,α-dimethylbenzyl)-1,3,4-oxadiazole-2-yl]carbonyl]-2-methylpropyl]acetamide,
(42) 2-[6-oxo-2-(4-fluorophenyl)-1,6-dihydro-1-pyrimidinyl]-N-[1-[[5-(α,α-dimethylbenzyl)-1,3,4-oxadiazole-2-yl]carbonyl]-2-methylpropyl]acetamide,
(43) 2-[6-oxo-2-phenyl-1,6-dihydro-1-pyrimidinyl]-N-[1-[[5-(α,α-dimethylbenzyl)-1,3,4-oxadiazole-2-yl]carbonyl]-2-methylpropyl]acetamide,
(44) Methoxycarbonyl-L-valyl-N-[(1S)-1-[[5-(t-butyl)-1,3,4-oxadiazole-2-yl]carbonyl]-2-methylpropyl]-L-prolinamide,
(45) Methoxycarbonyl-L-valyl-N-[(1S)-1-[[5-(α,α-dimethylbenzyl)-1,3,4-oxadiazole-2-yl]carbonyl]-2-methylpropyl]-L-prolinamide,
(46) Methoxycarbonyl-L-valyl-N-[(1S)-1-[[5-(α,α-dimethyl-3,4-methylenedioxybenzyl)-1,3,4-oxadiazole-2-yl]carbonyl]-2-methylpropyl]-L-prolinamide,
(47) 2-[5-methoxycarbonylamino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide,
(48) 1-[(3S)-(2-(t-butoxycarbonyl)-1,2,3,4-tetrahydroisoquinoline-3-yl]-N-[(1S)-1-[[5-(t-butyl)-1,3,4-oxadiazole-2-yl]carbonyl]-2-methylpropyl]carboxamide,
(49) 1-[(3S)-(2-(1-methylethoxycarbonyl)-1,2,3,4-tetrahydroisoquinoline-3-yl]-N-[(1S)-1-[[5-(t-butyl)-1,3,4-oxadiazole-2-yl]carbonyl]-2-methylpropyl]carboxamide,
(50) 1-[(3S)-(2-(imidazole-5-ylcarbonyl)-1,2,3,4-tetrahydroisoquinoline-3-yl]-N-[(1S)-1-[[5-(t-butyl)-1,3,4-oxadiazole-2-yl]carbonyl]-2-methylpropyl]carboxamide,
(51) 1-[(3S)-(2-[(1S)-1-(methoxycarbonylamino)-2-methylpropylcarbonyl]-1,2,3,4-tetrahydroisoquinoline-3-yl]-N-[( 1S)-1-[[5-(t-butyl)-1,3,4-oxadiazole-2-yl]carbonyl]-2-methylpropyl]carboxamide,
(52) 1-[(3S)-(2-[(1S)-1-(pyridine-3-ylcarbonylamino)-2-methylpropylcarbonyl]-1,2,3,4-tetrahydroisoquinoline-3-yl]-N-[(1S)-1-[[5-(t-butyl)-1,3,4-oxadiazole-2-yl]carbonyl]-2-methylpropyl]carboxamide,
(53) 1-[(2S)-1-(t-butoxycarbonyl)-2,3-dihydroindole-2-yl]-N-[(1S)-1-[[5-(t-butyl)-1,3,4-oxadiazole-2-yl]carbonyl]-2-methylpropyl]carboxamide,
(54) 1-[(2S)-1-(2-methylethoxycarbonyl)-2,3-dihydroindole-2-yl]-N-[(1S)-1-[[5-(t-butyl)-1,3,4-oxadiazole-2-yl]carbonyl]-2-methylpropyl]carboxamide,
(55) 1-[(2S)-1-(imidazole-5-ylcarbonyl)-2,3-dihydroindole-2-yl]-N-[(1S)-1-[[5-(t-butyl)-1,3,4-oxadiazole-2-yl]carbonyl]-2-methylpropyl]carboxamide,
(56) 1-[(2S)-1-[(1S)-1-(methoxycarbonylamino)-2-methylpropylcarbonyl]-2,3-dihydroindole-2-yl]-N-[(1S)-1-[[5-(t-butyl)-1,3,4-oxadiazole-2-yl]carbonyl]-2-methylpropyl]carboxamide,
(57) 1-[(2S)-1-[(1S)-1-(pyridine-3-ylcarbonylamino)-2-methylpropylcarbonyl]-2,3-dihydroindole-2-yl]-N-[(1S)-1-[[5-(t-butyl)-1,3,4-oxadiazole-2-yl]carbonyl]-2-methylpropyl]carboxamide,
(58) 2-[5-methoxycarbonylamino-6-oxo-2-phenyl-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide.

8. Prophylactic and/or therapeutic drugs described in claim 1 wherein a disease with the augmentative airway mucus secretion is asthma.
